# FASCICULE DE BREVET EUROPEEN

(11) **EP 4 638 774 B1**
(45) Date de publication et mention de la délivrance du brevet: **14.10.2026**
(21) Numéro de dépôt: 23834161.4
(22) Date de dépôt: 19.12.2023
(51) Int. Cl.: C12Q 1/02, B01L 3/00, G01N 33/50, G01N 33/569, G01N 33/543

(54) **DISPOSITIF POUR LA CARACTERISATION HOLISTIQUE DU COMPLEXE PEAU-MICROBIOTE ET METHODE DE RECOMMANDATION COSMETIQUE**
VORRICHTUNG ZUR GANZHEITLICHEN CHARAKTERISIERUNG DES HAUT-MIKROBIOTA-KOMPLEXES UND KOSMETISCHES EMPFEHLUNGSVERFAHREN
DEVICE FOR HOLISTIC CHARACTERISATION OF THE SKIN-MICROBIOTA COMPLEX AND COSMETIC RECOMMENDATION METHOD

(30) Priorité: 19.12.2022 FR 2213860
(43) Date de publication de la demande: 29.10.2025
(73) Titulaire: BYOME LABS, 28630 Le Coudray (FR)
(72) Inventeur: BERNARDI, Thierry, 63170 Pérignat-Les-Sarliève (FR); SUISSA, David, 94120 Fontenay-Sous-Bois (FR)
(74) Mandataire: IP Trust
(86) Numéro de dépôt international: PCT/EP2023/086672
(87) Numéro de publication internationale: WO 2024/133284

(56) Documents cités:
- WO-A1-2020/161238
- WO-A1-2022/121864
- US-A1- 2015 148 265
- US-A1- 2019 369 119
- BYRD ALLYSON L ET AL: "The human skin microbiome", NATURE REVIEWS MICROBIOLOGY, NATURE PUBLISHING GROUP, GB, vol. 16, no. 3, 15 January 2018 (2018-01-15), pages 143 - 155, XP037115228, ISSN: 1740-1526, [retrieved on 20180115], DOI: 10.1038/NRMICRO.2017.157
- HUANG RYAN YUKI ET AL: "Circulating Antibodies to Skin Bacteria Detected by Serological Lateral Flow Immunoassays Differentially Correlated With Bacterial Abundance", FRONTIERS IN MICROBIOLOGY, vol. 12, 10 November 2021 (2021-11-10), XP093077144, DOI: 10.3389/fmicb.2021.709562
- GOLEVA ELENA ET AL: "Skin tape proteomics identifies pathways associated with transepidermal water loss and allergen polysensitization in atopic dermatitis", JOURNAL OF ALLERGY AND CLINICAL IMMUNOLOGY, ELSEVIER, AMSTERDAM, NL, vol. 146, no. 6, 28 April 2020 (2020-04-28), pages 1367 - 1378, XP086384143, ISSN: 0091-6749, [retrieved on 20200428], DOI: 10.1016/J.JACI.2020.04.022

## Description

La présente invention concerne le domaine de la caractérisation du microbiote présent sur la peau humaine (ou « microflore » de la peau humaine). Elle concerne plus particulièrement un dispositif de révélation immunochromatographique du complexe peau-microbiote permettant de faire un relevé biologique rapide et significatif des deux membres de la symbiose peau-microbiote grâce à une caractérisation immunologique de marqueurs biologiques accessibles en surface de l'interactome microbiote-peau. Une Intelligence artificielle permet ensuite de conseiller le meilleur soin adapté d'une part au type de peau et d'autre part compatible avec son microbiote, réduisant les risques d'effets indésirables. De la même façon, en cas d'imperfection de la peau et/ou de déséquilibre du microbiote, la présente invention permet de recommander le meilleur soin correctif, soit garanti compatible avec le microbiote, soit capable de le réguler.

### Domaine de l'invention

Le domaine de l'invention est celui du conseil de soin cosmétique. Sans inférer dans le domaine de la Santé, un produit cosmétique doit pouvoir remplir son rôle de produit de soin pour maintenir ou améliorer l'apparence de l'utilisateur. Mis en contact avec les diverses parties superficielles du corps humain (épiderme, systèmes pileux et capillaire, ongles, lèvres et organes génitaux externes), il ne doit présenter aucun danger pour la santé. Dans un objectif de conseil personnalisé, la présente invention propose d'apporter une information sur la peau et le microbiote associé, correspondant à une symbiose, en considérant qu'un produit doit à la fois être adapté au besoin de la peau en hydratation, en hygiène, en protection, en modification d'aspect (teint, couleur...) ainsi qu'à la susceptibilité du microbiote. Par exemple en conseillant des produits contenant des principes actifs compatibles avec le maintien ou la régénération du microbiote, et en évitant des principes actifs déstabilisant ledit microbiote (favorisant une espèce microbienne pouvant induire un risque de prolifération opportuniste). Par extension, le domaine de l'invention peut s'appliquer au Diagnostic de Santé de la peau d'un patient, en permettant de consolider le diagnostic clinique relatif par exemple à l'état d'inflammation, de réaction allergique (résultat d'une réaction du système immunitaire) et au statut microbiologique (microbiote équilibré ou dysbiose avec forte présence d'un pathogène opportuniste). L'invention peut alors devenir un Dispositif Médical de Diagnostic *in vitro.* De la même façon, par extension, l'invention peut être utilisée pour réaliser un suivi de l'état de la peau sur une période donnée et suivre l'effet d'une routine cosmétique, mais aussi d'un traitement thérapeutique préventif et/ou curatif.

Le domaine de l'invention s'adresse à l'homme en tant qu'«hybride mammifère-microbes», un « super-organisme», notre microbiote excédant d'un facteur 10 le nombre de cellules composant notre corps. Le microbiote étant entendu-là comme réunissant l'ensemble des microorganismes au contact des cellules constitutives du corps humain : virus, phages, bactéries, levures, champignons... jusqu'aux acariens. Si l'on se restreint au microbiote bactérien, l'ordre de grandeur se ramène à un facteur 1,3. Cette situation de symbiose homme-microbes est commune à l'ensemble du monde animal et végétal : il a donc fallu que se mette en place tout au long de l'évolution une relation eucaryotes-procaryotes complexe, affinée, diversifiée, complexifiée par des pressions sélectives réciproques. Le rôle du microbiote est multiple : effet de barrière contre la colonisation par les agents pathogènes, effet de maturation sur le système immunitaire, mais aussi sur la peau elle-même, apport métabolique et nutritionnel majeur par la mise à disposition d'une capacité inexistante chez les mammifères d'hydrolyse des sucres végétaux complexes, de production de nutriments comme les acides gras à chaînes courtes et de certaines vitamines.

Le domaine de l'invention prend en considération de façon associée deux partenaires symbiotiques trop longtemps considérés de façon dissociée : l'organe « peau » et le microbiote, longtemps appelé « flore cutanée ».

### Le microbiote

La « flore cutanée » constitue la partie externe du microbiote de l'organisme humain (avec le microbiote du tube digestif -le plus important en biomasse-, et des cavités orales, nasales, auriculaires..., et vaginales chez la femme). Cette flore joue un rôle barrière vis-à-vis des agressions extérieures physiques, chimique et biologiques), et interagit avec l'organisme hôte en fonction des sites, de façon générale via les cellules du système immunitaire. De manière générale, la biodiversité bactérienne limite le risque de colonisation de la peau par une bactérie pathogène et constitue une protection contre l'inflammation, une réaction allergique de la peau.

En raison de la taille microscopique des organismes qui la composent, la microflore humaine est invisible à nos yeux. La peau d'un adulte héberge en moyenne 1 millions de bactéries par cm2 de peau, de plus de 500 espèces différentes. La peau supporte et entretient naturellement son propre « micro-écosystème ». Celui-ci se forme à la naissance, puis évolue jusqu'à la mort. Le microbiote cutané est installé physiquement à la surface, c'est un consortium de microbes organisés en biofilm, il s'alimente à la fois de molécules et de composés excrétés par la peau elle-même, et de composés sécrétés par des communautés de microorganismes plus ou moins symbiotes, principalement des bactéries organisées en biofilm. La composition du microbiote cutané varie selon les individus, leur âge (nouveau-né, adolescence, adulte, vieillesse), leur sexe (puberté, ménopause chez la femme), leurs activités, leur comportement et l'environnement. Le biofilm cutané se renouvelle constamment, pour s'adapter à la desquamation naturelle et à l'érosion différenciée de la peau, ainsi qu'aux pratiques d'hygiène corporelle (qui ne doivent pas être excessives pour protéger les fonctions « barrière » de la peau, ni réduire la diversité du microbiote qui protège de l'inflammation). De plus, il évolue avec l'âge. Chez l'enfant, le biofilm bactérien est différent selon le mode d'accouchement : par voie génitale, l'enfant sera colonisé par une communauté similaire au microbiote vaginal de la mère alors que par césarienne ce sera par une communauté similaire au microbiote cutané. Après 1 mois, les profils des communautés cutanées se modifient régulièrement pour que graduellement se mettent en place les spécificités taxonomiques (virus, bactéries, levures, champignons, et même acariens) des différents sites différenciés sur le corps humain : sites externes sur la peau (face, cuir chevelu...), les cavités (orale, nasale... vaginale), et sites internes (tube digestif principalement). Une étude récente (Huang, 2020) a même déterminé quelle région du corps (tube digestif, cavité orale, peau) du microbiome pourrait le plus précisément prédire l'âge et a constaté que la peau était la meilleure, donnant en moyenne des prédictions précises à 4 ans. L'importance du niveau de symbiose entre le microbiote et la peau selon l'âge est de plus en plus documentée (Kim, 2019 ; Trojahn, 2015), confirmant la nécessité de s'assurer d'une part que les produits cosmétiques appliqués sur la peau soient bien compatibles avec le microbiote résident, d'autre part qu'il peut être judicieux de suivre l'évolution de la symbiose homme-microbiote tout au long d'une vie.

Le microbiote joue un rôle dans l'odeur de la peau humaine, qui évolue aussi tout au long de la vie. La production des odeurs peut provenir de la dégradation des composés de la sueur tels que les acides gras volatiles ou les stéroïdes odorants (James *et al*., FEMS Microbiology Ecology, 2013), avec l'implication de certains genres bactériens comme *Corynebacterium* et de souches affiliées à *Streptococcus.* Participent aussi à l'apparition des odeurs corporelles des acides gras courts, tels que l'acide caprilique, caprique, valérianique ou l'acide propionique, produits de dégradation des acides aminés et des acides gras à longue chaîne carbonée issus du sébum par les bactéries du genre *Brevibacterium* et *Propionibacterium* ; l'acide propionique donne l'odeur de noisette de la peau à basse concentration (typiquement après une douche) ou l'odeur de fromage de la peau sale à forte concentration. Ces odeurs corporelles varient en fonction du sexe, de l'âge ou du cycle menstruel.

Le biofilm cutané joue un rôle tantôt positif, tantôt négatif. L'installation d'un microbiote commensal sur la peau correspond à une colonisation, des groupes de microorganismes ayant un rôle protecteur contre d'autres microorganismes pathogènes, ce qui correspond à une infection, peuvent être identifiés par des techniques d'analyses par séquençage nouvelle génération. Il a été décrit par exemple dans les cas d'infections de plaies (cas extrême où la peau présente une discontinuité, une brèche, au lieu d'une surface homogène et continue) des groupes de bactéries appelés « pathogroupes » (*Proteus, Morganella, Anaerococcus et Peptoniphilus*) dont la présence est corrélée avec des plaies se dégradant, ne cicatrisant pas (Dunyach-Rémi et al. 2020). A contrario, toujours dans les cas d'infections de plaies, des bactéries ont été identifiées comme exerçant une activité antimicrobienne contre des pathogènes (Nakatsuji et al. , 2017), ou réduisant la virulence de pathogènes (Ngba Essebe et al., 2017), protégeant ainsi l'hôte et à l'origine d'une notion de « flore positive ».

Le microbiote résident sur la peau est invisible en dehors des cas où il provoque des réactions cutanées du système immunitaire, par exemple des boutons d'acné liés à la prolifération de la bactérie *Cutibacterium acnes,* des rougeurs liées à un état inflammatoire d'eczéma ou dermatite atopique exacerbées par l'installation de la bactérie *Staphylococcus aureus...* Il existe aussi une corrélation entre des taches sur la peau et la présence de certains microorganismes, par exemple des genres *Kocuria* et *Aerococcus* (Zanchetta et al., 2022).

La microscopie a longtemps été la principale voie d'observation du microbiote cutané. Les progrès des techniques biomoléculaires ont ouvert de nouveaux champs d'étude via la génomique, la génétique moléculaire, la métagénomique, le séquençage haut débit et la « culturomique ». Cette approche, via le séquençage direct de l'ADN présent dans l'échantillon, permet une description génomique du contenu de l'échantillon, mais aussi un aperçu du potentiel fonctionnel d'un environnement. Pour étudier des échantillons métagénomiques, les biologistes recourent aux technologies de séquençage haut-débit. Les séquences d'ADN obtenues sont ensuite analysées par des techniques bioinformatiques. Les études métagénomiques nécessitent une logistique d'acheminement de l'échantillon prélevé sur la personne en général avec un simple coton-tige vers le laboratoire d'analyse qui lui-même doit être équipé d'un parc de matériel pour l'extraction du matériel génomique (ADN) et son séquençage.

Pour révéler la présence de ces microorganismes installés en biofilm et caractéristiques d'un état de la peau, il est aussi possible d'utiliser les marqueurs biologiques, des molécules présentes à la surface des membranes, des molécules d'adhésion aux cellules de la peau, des molécules sécrétés (exopolysaccharides, ADN, protéines...) pour constituer la matrice du biofilm. Ces marqueurs biologiques peuvent être prélevés sur la personne et immédiatement analysés avec des techniques d'immunochromatographie en flux latéral (Lateral Flow Immuno-Assay, LFIA).

Ainsi, les microorganismes pathogènes peuvent être distingués des commensaux en se basant sur les marqueurs moléculaires associés aux agents pathogènes (en anglais : pathogen-associated molecular patterns, PAMP). Ces marqueurs dits « de virulence » sont les plus documentés, décrits dans la littérature. Ce sont des molécules sécrétées, par exemple la protéine Leucocidine de Pantone-Valentine de *Staphylococcus aureus,* mais aussi, avec un degré moindre de virulence, des porphyrines produites par *Cutibacterium acnes,* des molécules fluorescentes visibles sous éclairage Ultra-Violet (lampe de Wood), qui induisent l'agrégation de *Staphylococcus aureus* et l'expression de molécules pro-inflammatoires par les kératinocytes (IL-6, IL-8, prostagladinesE2, TBF-alpha...).

D'autres marqueurs sont caractéristiques de l'installation naturelle en biofilm des microorganismes à la surface de la peau et dans le conduit du follicule pilosébacé (voir plus loin). De façon générale, les microorganismes interagissent avec une famille de protéines de la peau appelées les « extracellular matrix » (ECM)-components ou composés de la matrice extra-cellulaire : plasminogène, fibronectine, laminine ou mucine. Pour cela, des protéines d'adhésions sont utilisées avec, pour les bactéries gram positive (simple membrane) : des adhésines de la famille Bap, des pili de type 4 (important pour l'installation précoce du biofilm), des SAATs (Self-associating autotransporters, promoteur de l'agrégation entre bactéries porteuses de SAATs), des intimines/invasines... ; et pour les bactéries gram négatives (double membrane - LPS) : des adhésines de la famille Bap exhibant un domaine C-terminal LPXTG, des IMPs (Inner-membrane proteins), des pili de type 3 et 4...

Par exemple pour *Cutibacterium acnes,* bactérie commensale pouvant devenir un pathogène opportuniste et provoquer l'acné. Cette bactérie peut former un biofilm soit au niveau des follicules pilosébacés soit au niveau des kératinocytes, grâce à des protéines présentes à la surface de la membrane bactérienne, comme des « CAMP factors (Christie-Atkins-Munch-Petersen factors), des sialidases, des dermatan-sulphate adhesines, des endoglycocéramidases, des chaperonines GroEL », un « SH3 domain-containing lipoprotein », une protéine de type pili/fimbriae Flp, et surtout une protéine DsA1, faisant partie de la famille des MSCRAMMs (molecular surface components recognizing adhesive molecules of the matrix), très immunogène et provoquant une forte réponse immunitaire chez les personnes atteintes d'acné.

Le genre bactérien Staphylococcus joue un rôle majeur au sein du microbiote de la peau avec l'espèce *Staphylococcus epidermidis* qui est commensale et rarement pathogène, et l'espèce *Staphylococcus aureus,* commensale avec un portage nasal sain chez 30% de la population, pouvant devenir pathogène en particulier dans les cas de Dermatite Atopique ou eczéma. Le genre Staphylococcus utilise aussi pour s'installer en biofilm sur la peau des MSCRAMMs comme les protéines de la famille Clf-Sdr, avec la Bbp (bone sialoprotein-binding protein), la FnBPs (fibronectin-binding proteins), et la CNA (collagen adhesion), et récemment a été identifiée pour *S. epidermidis* la protéine SesJ (Arora 2020). Pour *S. aureus,* c'est l'aureusimine (phevalin) qui semble être un marqueur du phénotype biofilm. Notons que le genre *Staphylococcus* sécrète aussi une matrice extracellulaire « polysaccharidique intercellular adhesin » (PIA). Les autres principaux représentant du microbiote cutané sont (Byrd et al., 2018) soit communs à tout type de peaux : *Corynebacterium tuberculostearicum, Malassezia Globosa* ; soit plutôt caractéristiques des peaux grasses, ou sèches ou humides : *Staphylococcus capitis, Staphylococcus hominis, Streptococcus mitis, Streptococcus oralis, Micrococcus luteus*, , *Corynebacterium simulans;* ou alors des microorganismes caractéristiques des peaux agées : *Dermacoccus, Actinomyces*; des peaux jeunes avec les *Bacteroidetes* tels: *Bacteroides, Alistipes, Prevotella, Porphyromonas, Sphingobacterium,* ou avec les Firmicutes *tels* : *Lactobacillus, Aerococcus, Oscillospira, Ruminococcus.*

Les organismes souvent considérés comme non pathogènes peuvent causer une infection chez des hôtes dont l'immunité est affaiblie ou qui ont récemment reçu un traitement antimicrobien. Lorsque les réponses immunitaires sont altérées, comme c'est souvent le cas de personnes atteintes de diabète, ils peuvent ne pas prévenir la colonisation par des bactéries pathogènes dans le tissu lésé. Dans les plaies chroniquement infectées, de nombreuses bactéries forment un biofilm, dans lequel ils se fixent et se développent de manière irréversible sur une surface, produisent des polymères extracellulaires qui facilitent la formation de la matrice et modifient leur phénotype.

Les bactéries les plus communes sur la peau humaine sont de type Gram-positives et appartiennent principalement à 5 genres. On trouve les bactéries suivantes :
- *Staphylococcus,* souvent opportunistes comme *Staphylococcus epidermidis,* qui constitue plus de 90 % de la flore résidente aérobie présente sur le *stratum corneum.* D'autres staphylocoques qui ont été trouvés dans le biofilm cutané sont *Staphylococcus aureus* (commun, trouvé par exemple en portage asymptomatique dans les cavités nasales dans 30 % des cas) et *Staphyloccocus hominis* ;
- *Corynebacterium*
- *Propionibacterium* (par exemple *Cutibacterium acnes, Propionibacterium granulosum, Propionibacterium avidum*). *Cutibacterium acnes* produit des acides gras issus de la lipolyse du sébum. Ce faisant elle acidifie le milieu cutané, ce qui inhibe la croissance des colonies de *Streptococcus pyogenes ;*
- *Lactobacillus* ;
- *Streptococcus.*

De façon générale, 3 bactéries sont majoritairement rencontrées : 1) *Staphylococcus epidermidis,* bactérie commensale de la peau avec un rôle barrière face au *Staphylococcus aureus ; 2) Cutibacterium acnes,* commensal de la peau, en général anodin, à l'origine cependant d'épisodes d'acné selon l'âge (puberté), l'imprégnation hormonale, une immuno-dépression (transitoire, iatrogène...) ; 3) *Staphylococcus aureus,* bactérie commensale, portée de façon asymptomatique chez 30% des individus (portage nasal), à l'origine de pathologies diverses au niveau de la peau (impétigo, dermatite atopique...).

Pour donner un aperçu général du domaine de l'invention, on peut indiquer que les affections cutanées sont les grandes oubliées de la santé publique car elles engagent rarement le pronostic vital. Elles affectent pourtant la qualité de vie de 16 millions de Français avec des répercussions psychologiques souvent sous-estimées, liées aux imperfections de la peau (tâches, rougeurs, boutons...) que les personnes atteintes atténueront ou masqueront par l'utilisation de produits cosmétiques.

En France, 1 personne sur 3 souffre d'une maladie de peau, selon une vaste étude épidémiologique (étude "Objectifs Peau" réalisée en 2016 sur un échantillon représentatif de 20 012 personnes âgées de plus de 15 ans) réalisée par la Société Française de Dermatologie (SFD). Et 80% des patients concernés en cumulent même deux. Et les femmes sont davantage touchées que les hommes : 33% d'entre elles ont une pathologie dermatologique, contre 28% de la gente masculine.

Ces chiffres alarmants sont bien supérieurs aux estimations précédentes. Le stress de la vie moderne et la pollution croissante jouent sûrement un rôle non négligeable dans l'augmentation du nombre de troubles cutanés.

Parmi les affections cutanées les plus fréquentes, l'acné arrive en tête de liste (3,3 millions de Français en souffrent même une fois la puberté passée, suivie de l'eczéma (2,5 millions de malades) et du psoriasis (2,4 millions). Les maladies du cuir chevelu (hors pelade), les mycoses et les maladies des ongles arrivent derrière, avec respectivement 2,3 millions, 2,2 millions et 2,1 millions de Français touchés.

Pour toutes ces affections se combinent des critères individuels affectant l'état de la peau (susceptibilités génétiques - mutations de gènes codant pour des protéines de la peau comme la filaggrine, système immunitaire défaillant... -, mode de vie - stress, cabines UV... -, alimentation...) et des critères affectant le microbiote (naissance par césarienne, hygiène, environnement...). Concernant l'effet du rayonnement Ultra-Violet, une étude pilote (Burns, 2019) montre une augmentation du phylum *Cyanobacteria,* une diminution des familles *Lactobacillaceae* et *Pseudomonadaceae,* une réactivité des différentes espèces selon les UVA et les UVB, et un potentiel rôle protecteur et anti-inflammatoire des *Lactobacillaceae.* Là aussi un test permettant d'évaluer l'effet de l'exposition aux UV sur la symbiose homme-microbiote peut permettre d'améliorer le conseil d'une part en produits réhydratant la peau et régénérant/rééquilibrant le microbiote (conseil curatif) et d'autre part en crème solaire protectrice (conseil préventif).

C'est la raison pour laquelle les sociétés cosmétiques s'intéressent depuis quelques années à la connaissance du microbiote cutané pour offrir à leurs clients des compositions adaptées et personnalisées permettant de préparer des crèmes actives à la fois adaptées à la physiologie de la peau (grasse, sèche... irritée, avec un certain niveau d'inflammation...) et aussi les mieux adaptées à l'état du microbiote cutané de chaque client, à une période donnée car on s'est aperçu que les effets revendiqués des crèmes anti-rides et anti-âge et de différents produits cosmétiques pour les soins de la peau ont des effets et une efficacité qui ne sont pas similaires sur tous les individus ni même, pour un même individu, à toutes les périodes. Différentes personnes et différents types de peau réagissent différemment aux produits cosmétiques, d'où le besoin de dispositifs qui peuvent déterminer les effets ou la réactivité d'un individu à un type particulier de produit de soin de la peau. Il est pour cela nécessaire de disposer d'une méthode d'analyse fiable et simultanée de l'état de la peau et du microbiote cutané.

### La peau

Le deuxième membre de la symbiose concerné par le domaine de l'invention est l'organe « Peau » : la partie exposée de la peau s'appelle la couche cornée (*stratum corneum*), une superposition de cellules anucléées et complètement kératinisées, les cornéocytes, formant des lamelles très allongées (ROBERT et al. Dermopharmacologie, Edisem, 1985). Son épaisseur est d'environ 10 µm sauf au niveau de la paume des mains et de la plante des pieds où il est environ 10 fois plus épais.

Cette couche cornée est le produit final externe de cet organe, la peau, couvrant en moyenne 1,5 à 2m². La peau est structurée en 3 couches de tissus : l'hypoderme (la plus profonde), le derme (intermédiaire) et l'épiderme (la plus superficielle).

L'hypoderme forme la couche la plus profonde de la peau. C'est un tissu conjonctif richement vascularisé qui contient beaucoup de tissu adipeux, lui-même constitué de cellules nommées adipocytes. Cette couche est dotée d'une grande élasticité capable de bien absorber les chocs et de plus, elle permet d'isoler le corps thermiquement.

Le derme correspond à la couche intermédiaire. C'est aussi un tissu conjonctif qui va venir soutenir l'épiderme. Il est parcouru par un riche réseau de vaisseaux capillaires et est essaimé de nombreuses terminaisons nerveuses. Il se divise en deux : le derme papillaire (superficiel) et le derme réticulaire (profond et moyen). Les fibroblastes disséminés dans la couche dermique synthétisent les fibres de collagène et les fibres élastiques qui baignent dans une sorte de gel qu'on appelle la matrice extracellulaire constituée d'eau et de glycoprotéines. Celle-ci joue le rôle de réservoir hydrique qui peut être affecté par l'environnement (temps sec en hiver), ou la surexposition aux rayonnements Ultra-Violets (bains de soleil, cabines UV...) provoquant une évaporation accrue. On y trouve aussi des cellules du système immunitaire telles que les macrophages, les cellules dermiques dendritiques, les mastocytes, les lymphocytes et des cellules en cours de maturation. La riche vascularisation de cette couche supporte plusieurs fonctions. Elle permet à la première ligne de défense de l'organisme de répondre efficacement à tout signal de danger, en particulier avec le microbiote lors d'une dysbiose ou lorsqu'une plaie crée une brèche dans la couche supérieure externe protectrice. L'épiderme dépourvu de capillaires y puise l'énergie et les nutriments nécessaires pour assurer son activité cellulaire. Elle participe de façon importante à la thermorégulation à côté des glandes sudoripares. Les glandes sudoripares et les follicules pilosébacés sont des annexes épidermiques implantées dans le derme.

L'épiderme est la structure la plus externe, non vascularisée, elle se divise en 5 couches superposées (interne à externe) :
- La couche basale (*stratum basale*) permet la régénération de la peau grâce à des divisions cellulaires (la quasi-totalité des cellules sont en mitose) qui vont permettre aux cellules produites de migrer en direction de la couche la plus externe. Cette couche est composée en majeure partie de kératinocytes.
- La couche épineuse (*stratum spinosum* ou couche de Malpighi) est la couche la plus épaisse de l'épiderme. Elle est composée de kératinocytes.
- La couche granuleuse (*stratum granulosum*), plus mince, est formée de kératinocytes qui rentrent en apoptose. Leur cytoplasme comprend moins d'organites cytoplasmiques et de chromatine nucléaire. Les cellules sont plus aplaties et se caractérisent par la présence de grains de kératohyaline et de granules lamellaires (ou corps d'Odland).
- La couche claire (*stratum lucidum*) possède des cellules anucléées (leurs noyaux ont disparu). Les grains de kératohyalines se transforment en une protéine appelée filaggrine.
- La couche cornée (*stratum corneum*), la plus superficielle, se compose de cellules mortes, de forme aplatie, les cornéocytes. Elles sont dites mortes mais elles demeurent biologiquement actives. Elles ont perdu tous leurs organites, remplacés par des filaments denses de kératine et elles sont liées entre elles par un ciment interlipidique formé d'acide gras, de cholestérol et de céramides ainsi que des cornéodesmosomes. Ce ciment se forme à partir des corps d'Odland que l'on retrouve dans les couches précédentes. Ces cellules jointives et bien tassées forment un revêtement imperméable à la peau et jouent un grand rôle de défense.

Le processus de kératinisation assure un renouvellement continu de la peau, de la couche basale jusqu'à la couche cornée, en 3 - 4 semaines. Les différents éléments la composant vont se retrouver à la surface et vont constituer la surface au niveau de la couche cornée sur laquelle le microbiote s'installe en biofilm. Les marqueurs biologiques de l'état de la peau vont s'y retrouver et permettre d'évaluer si elle présente un état sain, une imprégnation hormonale (puberté, cycle menstruel...), une inflammation (d'origine endogène ou exogène), une réaction allergique, une agression (physique : rayonnements Ultra-Violets, pollution ; chimique : produits d'hygiène, produits cosmétiques ; mécanique : abrasion lors d'un gommage, d'une exfoliation ou d'un peeling...).

La couche cornée est le premier rempart entre le corps humain et le monde extérieur, elle concentre le rôle de barrière contre la pénétration d'agents extérieurs, permettant d'éviter le passage d'agents délétères, microbiens ou chimiques à l'intérieur de notre corps. En cas de défaillance de ce premier niveau de protection, un autre mécanisme de protection est mis en jeu faisant intervenir les cellules du système immunitaire.

La couche cornée est le premier niveau de l'interactome, en contact direct, où le microbiote va s'installer en biofilm en utilisant des protéines d'adhésion et sécréter des exopolysaccharides lui permettant de persister sur la peau.

Lorsque la barrière physique de la couche cornée n'opère plus parce qu'éliminée physiquement par abrasion, par exemple en surface suite à un gommage ou une exfoliation prononcé ou un peeling, ou plus profondément en cas de plaie traumatique ou chirurgicale, c'est le système immunitaire cutané qui intervient. Il a pour but de protéger l'hôte et de restaurer si besoin l'intégrité de la peau. Il se divise en deux types : en premier lieu l'immunité innée, ensuite l'immunité adaptative ou spécifique. Dans les deux cas, les premiers gardiens sont les kératinocytes car ce sont les plus nombreux (90% des cellules de la peau). Ils jouent le rôle de sentinelles immunitaires et reconnaissent les agents étrangers grâce à des récepteurs PRR (pattern recognition receptor) de la famille des Toll-like, qui, une fois la reconnaissance réalisée, synthétisent des médiateurs chimiques (cytokines et chimiokines). Ils initient la cascade inflammatoire. D'autre part, les kératinocytes sont aussi capables de produire des peptides antimicrobiens qui vont inhiber des microorganismes comme *Staphylococcus aureus, Candida Albicans.*

L'épiderme contient 2 types de cellules immunitaires. Les cellules dendritiques, ou cellules de Langerhans, formées de pseudopodes qui vont leur permettre d'accrocher l'élément pathogène et de le phagocyter. On appelle ces cellules des CPA : cellules présentatrices d'antigènes. Une fois l'élément pathogène ou antigène phagocyté, la cellule dendritique va sécréter des médiateurs chimiques (prostaglandines ou chimiokines) qui vont entraîner une vasodilatation locale et vont donc augmenter l'afflux sanguin et le recrutement local d'autres cellules de l'immunité comme les polynucléaires et les macrophages. Les cellules immunitaires du derme (cellules dendritiques dermiques, macrophages, mastocytes) sont également mobilisées sur le site concerné. L'épiderme héberge aussi des lymphocytes T qui ont été activés au niveau des ganglions lymphatiques grâce aux CPA. Ainsi, après l'activation des CPA, s'ensuit une cascade de multiplications, de différenciations qui doit aboutir *in fine* à une destruction des agents pathogènes. Ces marqueurs biologiques peuvent être utilisés pour évaluer l'état de la peau en surface, en particulier le niveau d'inflammation, qui peut être chronique et devient de plus en plus critique avec l'âge, on parle alors « d'inflammaging ». De façon plus exhaustive, les marqueurs biologiques de l'inflammation de la peau sont les interleukines IL-1beta, IL-4, IL-6, IL-8, IL-11, IL-12, le Facteur Nécrosant de Tumeur (en anglais Tumor-Necrosis-Factor-alpha, TNF-alpha), l'Interféron-gamma (IFN-gamma), le facteur stimulant les colonies de granulocytes et de macrophages (en anglais Granulocytes Macrophages Colony-Stimulating Factor, GM-CSF), le Facteur de Croissance Transformant-beta (en anglais Transforming Growth Factor-beta, TGF-beta). Sans être directement considérées comme marqueurs de l'inflammation, il faut aussi mentionner les Immunoglobulines E (IgE) dont le niveau sérique est élevé dans les cas d'eczémas - dermatites atopiques, et qui sont à l'origine de la majorité des réactions allergiques dites IgE-dépendantes.

D'autres cellules de la peau interviennent dans le maintien d'un état sain face aux agressions du soleil ou à l'exposition aux radiations Ultra-Violettes (par exemple en cabines utilisant des lampes UV). Ce sont les mélanocytes présents dans la peau qui jouent un rôle de protection contre les radiations ultra-violettes (UV). Lors de l'exposition de la peau aux UV, les kératinocytes sécrètent l'hormone mélanotrope ou a-MSH (α-mélanocyte stimulating hormone) par maturation d'une pro-hormone, la POMC (proopiomélanocortine). Cette hormone a-MSH va venir s'attacher à un récepteur membranaire appelé MCR (melanocortin receptor) exprimé par les mélanocytes et enclencher la biosynthèse de mélanine, à l'origine du bronzage de la peau. Le matériel génétique des cellules épidermiques soumises aux radiations UV devient ainsi protégé.

La description de l'organe « peau » se complète avec les structures annexes incluses au sein des différentes couches : les poils et les glandes sébacées et sudoripares.

Les poils couvrent presque intégralement le corps humain avec des tailles variables. Ils jouent un rôle très important dans la thermorégulation. Ils sont ancrés au niveau du derme dans ce qu'on appelle le follicule pileux.

La glande sébacée appendue au poil est une glande intradermique. Elle synthétise le sébum, constituant majeur avec la sueur du film hydrolipidique qui protège la peau et évite son dessèchement. Cette glande interagit avec le système immunitaire avec ensuite un impact sur le microbiote comme nous le verrons plus loin.

La glande sudoripare sécrète la sueur et, par ce processus, permet à l'organisme de lutter contre la chaleur. En effet lorsque la température du corps s'élève par exemple pendant une séance de sport ou quand on est malade, la sueur émise s'évapore à la surface de la peau : ce mécanisme élimine des calories et contribue avec la vasodilatation à refroidir le corps.

Parmi ces annexes, les glandes sébacées jouent un rôle central en conditionnant le pH de la peau, son caractère plus ou moins occlusif et donc l'accès à l'oxygène (impact sur les capacités aérobies ou anaérobies des microorganismes du microbiote), des effets antioxydant, des effets pro et/ou antimicrobiens (variables en fonctions des espèces de microorganismes), et le transport d'hormones/phéromones. Le sébum est composé de triglycérides, diglycérides, d'acides gras libres, d'esters de cire, de squalènes, et de cholestérols.

### La symbiose peau-microbiote

La symbiose homme-microbiote au niveau de la peau est d'autant plus complexe qu'il a été récemment décrit que des cellules immunitaires dites « lymphoïdes innées » (Innate Lympoid Cells, ILC) jouent un rôle régulateur sur les glandes sébacées (Kobayashi et al., 2019). Ces ILCs (en produisant des « TNF receptor ligands ») limitent la croissance des glandes sébacées. En cas de déficience de ces ILCs, on observe une hyperplasie des glandes sébacées ayant pour conséquence une augmentation de la production de lipides antimicrobiens, en particulier l'acide palmitoléique et l'acide oléique, avec un impact sur l'équilibre du microbiote cutané. En effet, l'acide palmitoléique inhibe la croissance des cocci aérobies gram positives telles *Staphylococcus aureus* et *Staphyloccoccus xylosus,* et pas la croissance d'anaérobies gram négatives comme les espèces *Bacteroides.* Associé à la surproduction d'acide oléique, cela créé un film occlusif, c'est-à-dire des conditions anaérobies en défaveur des cocci gram positives. Ceci peut conduire à ne pas recommander des produits cosmétiques contenant ces deux acides gras dans les cas où des marqueurs biologiques corrélés avec le profil avec « peau grasse + présence élevée de *Staphylococcus* » auront été caractérisés avec le dispositif de révélation immunochromatographique objet du présent brevet.

La symbiose homme-microbiote est aussi organisée à d'autres niveaux que celui de l'épiderme-stratum corneum mais aussi de façon plus large en fonction de différents facteurs endogènes comme la région corporelle (zone T du visage, cuir chevelu, aisselles..., l'âge, l'imprégnation hormonale (puberté, cycle menstruel...), une inflammation (d'origine endogène - dermatite atopique, psoriasis...-, ou exogène - contact avec un produit irritant -), une agression (physique : rayonnements Ultra-Violets, pollution ; chimique : produits d'hygiène, produits cosmétiques ; mécanique : abrasion lors d'un gommage ou exfoliation ou d'un peeling...).

Les régions corporelles sont segmentées en trois zones caractérisées par des paramètres physicochimiques différents :
- Les zones humides :
   Les zones dites « humides » sont caractérisées par une forte densité de glandes sudoripares. L'humidité est due à la sécrétion de sueur par ces glandes. La sueur est composée principalement d'eau, de sels minéraux, d'acide urique et d'urée. Les zones concernées du corps humain sont : le creux axillaire, le périnée, les plis interdigitaux, les paumes des mains et les aisselles au niveau desquelles on relève une forte colonisation par les micro-organismes : 10⁵ -10⁸ bactéries/cm².
- Les zones lipidiques :
   Les zones dites « lipidiques » sont liées à une forte présence de glandes sébacées. Ces glandes secrètent le sébum qui s'écoule vers la surface de la peau sous forme de film lipidique. La tête, notamment avec les joues, le front, le nez, le menton, le tronc et le haut du dos, constituent les principales zones lipidiques dont la concentration en micro-organismes est de l'ordre de 10⁶ à 10⁷ bactéries/cm².
- Les zones sèches :
   Les zones dites « sèches » sont pauvres en glandes sudoripares et sébacées et contiennent donc moins de sécrétions de sueur et de sébum.

Le dos des mains et la face externe des membres sont les principales zones sèches. Ces dernières ont la concentration en micro-organismes la plus faible : 10³-10⁴ bactéries/cm².

Le pH de la peau est de 5,5 en moyenne. Il est dû au film hydrolipidique qui recouvre toute la surface de la peau, produit des sécrétions des glandes sudoripares et des glandes sébacées. L'acidité relative de ce film permet notamment d'assurer une protection vis-à-vis des germes pathogènes. Le pH cutané s'avère être un paramètre très important à maintenir pour l'homéostasie cutanée et l'équilibre du microbiote installé en biofilm. Au cours de nombreuses pathologies, eczéma - dermatite atopique, psoriasis, acné..., il se trouve d'ailleurs modifié et il a été documenté une altération du pH dans des cas de dermatite atopique corrélée à la présence de *Staphylococcus aureus* (Rippke et al., 2004).

### ETAT DE LA TECHNIQUE

On connaît dans l'état de la technique différentes solutions concernant l'analyse du microbiote cutané.

Le document WO 2014184151 A1 décrit un dispositif de diagnostic « point of care » basé sur la technologie d'analyse à flux latéral et permet une analyse non invasive de facteurs sécrétés et diffusibles à partir de la surface de la peau. Ce document décrit en particulier un kit de diagnostic pour détecter la présence ou la quantité d'un ou plusieurs analytes dans un échantillon à tester prélevé sur une surface cutanée d'un mammifère, caractérisé en ce que le kit de diagnostic comprend :
a) un insert séparé pour un dispositif à écoulement latéral comprenant une membrane, la membrane ayant une épaisseur, une largeur et une longueur définies, éventuellement fixée à un support rigide, et l'insert séparé étant configuré pour obtenir l'échantillon à tester (analytes),
b) un dispositif de dosage à écoulement latéral configuré pour accepter l'insert séparé, et
c) un élément de fixation configuré pour attacher de manière amovible l'insert séparé à la surface de la peau d'un mammifère.

La demande de brevet US2022/0178943 propose un kit pour détecter la présence ou la quantité d'un ou plusieurs analytes à tester dans un échantillon à tester obtenu à partir d'une surface cutanée d'un mammifère, le kit comprenant :
a) un dispositif d'analyse à flux latéral (LFIA) comprenant une cassette comprenant un ou plusieurs éléments poreux formant un ensemble de supports poreux, dans lequel ladite cassette est configurée pour recevoir et maintenir un tampon de prélèvement d'échantillon, dans lequel ledit tampon de collecte d'échantillons est configuré pour être en contact avec ledit ensemble de supports poreux lorsque ledit tampon d'échantillon est inséré dans ladite cassette,
b) un emballage-coque, dans lequel ledit emballage-coque contient une solution tampon, dans lequel ladite cassette est configurée pour recevoir ledit emballage-coque, et
c) un tampon de collecte d'échantillon configuré pour être utilisé pour collecter ledit échantillon de test.

La demande EP3691788A1 concerne également un kit de diagnostic pour détecter la présence ou la quantité d'un ou plusieurs analytes de test dans un échantillon de test obtenu à partir d'une surface cutanée d'un mammifère, le kit de diagnostic comprenant :
a) un tampon séparé configuré pour être utilisé pour collecter ledit échantillon de test, ledit tampon comprenant un tampon de prélèvement d'échantillon attaché à un élément de support,
b) un dispositif d'analyse à flux latéral comprenant un ou plusieurs éléments poreux, dans lequel ledit dispositif d'analyse à flux latéral est configuré pour accepter et maintenir ledit écouvillon séparé, dans lequel ledit tampon de prélèvement d'échantillon est configuré pour faire partie d'un ensemble de support poreux lorsque l'écouvillon séparé est inséré dans ledit dispositif d'analyse à écoulement latéral.

La demande WO2019025610 décrit des kits et des procédés de diagnostic basés sur des dispositifs de dosage à écoulement latéral pour détecter la présence ou la quantité d'un ou de plusieurs analytes d'essai dans un échantillon d'essai prélevé sur la peau d'un mammifère.

Les demandes WO2020/161238 et US2015/148265 décrivent des dispositifs de révélation immunochromatographique d'échantillon de peau.

### Inconvénients de l'état de la technique

Il existe soit des solutions d'analyses basées sur des techniques de biologie moléculaire très précises mais longues et compliquées à mettre en œuvre, et chères, soit des solutions d'analyse à flux latéral qui ont uniquement un ou 2 marqueurs, et qui ne sont pas adaptés à la caractérisation de la peau. Les solutions de LFIA binaires permettent uniquement de révéler la présence ou l'absence de biomarqueurs.

Les méthodes utilisées pour récupérer les quantités d'analytes prélevées avec les solutions de l'art antérieur (coton-tige ou swab, bandes adhésives D-Squame ou Sebutape...) et les performances des tests immunochromatographiques à flux latéral nécessitant au minimum de l'ordre de 10⁴ analytes par millilitres, on observe le recours à des solutions d'analyse génomique avec une étape d'amplification de l'ADN pour obtenir des résultats significatifs.

Ces solutions ne sont pas totalement satisfaisantes car elles nécessitent après le prélèvement, des interventions complexes (extraction ADN, mise en œuvre de PCR, Q-PCR ou séquençage...), dans un laboratoire de biologie et un personnel expérimenté dans l'utilisation d'équipements destinés à la génomique.

Elles impliquent pour cela une logistique lourde et des délais de traitement entre le prélèvement et l'analyse génomique qui sont de l'ordre de plusieurs jours à plusieurs semaines et présentent en outre un coût relativement élevé.

En conséquence, le nombre d'études permettant d'évaluer l'impact sur la peau et son microbiote de produits de soins cosmétiques ou d'hygiène appliqués sur tout ou partie du visage (ou du reste du corps) reste très limité à ce jour (par exemple, selon le site clinicaltrial.gov, en 2017-18, l'étude « The Effect of Skin Cleansers on the Skin Surface Microbiome » dont le sponsor était Mundipharma Manufacturing Pte Ltd a inclus 12 volontaires seulement, avec analyse de leur microbiome cutané par Next Generation Sequencing, pour comparer les effets de 3 produits : 1) 7.5% Povidone-lodine, 2) 4% chlorhexidine skin cleanser, et 3) Plain non antibacterial soap.

In fine, les propositions de tests ou analyses du « microbiome » par métagénomique conduisent à générer sur une application smartphone associée un listing de microorganismes identifiés à titre documentaire. L'objectif final de la démarche consistant à conseiller des produits cosmétiques ou d'hygiène, qui s'inspirent des renseignements collectés via l'application smartphone lors de la prise de commande du test « microbiome de la peau » (photo du visage permettant d'évaluer des paramètres tels que rides, rougeur..., renseignements déclaratifs comme âge, poids, taille, consommation de tabac, d'alcool, estimation de l'état de la peau -irritée, sèche, grasse-, déclaration de pathologies -acné, eczéma, dermatite atopique, psoriasis...-, etc).

### Solution apportée par l'invention

L'invention vient remédier aux inconvénients de l'art antérieur en proposant une solution
- Rapide
- Peu chère
- Comprenant suffisamment d'éléments pour caractériser la peau et
- Permettant plusieurs niveaux de lectures des biomarqueurs pour donner suffisamment d'information pour faire une recommandation cosmétique.

Ainsi, la présente invention concerne un dispositif de révélation immunochromatographique du complexe peau-microbiote comprenant un support sur lequel est aménagé un puits de transfert destiné à recevoir une solution comprenant les constituants du complexe peau-microbiote, ledit puits débouchant sur une pluralité de N zones de révélation portant chacune (i) un réactif de détection immunologique et (ii) un réactif de capture immunologique, et ledit complexe peau-microbiote étant constitué par des cellules de la peau et des microorganismes et autres éléments constitutifs du microbiote cutané, ledit dispositif comprenant :
∘ un nombre de zones de révélation N, N étant égal ou supérieur à 5
∘ au moins un réactif de détection immunologique spécifique d'un biomarqueur de cellules de la peau et au moins un réactif de détection immunologique spécifique d'un biomarqueur du microbiote cutané
∘ lesdits réactifs de détection immunologique comprennent chacun un anticorps spécifique d'un biomarqueur de cellules de la peau ou du microbiote cutané, conjugué à un système de révélation colorimétrique
∘ lesdits réactifs de capture comprennent chacun un anticorps, fixé audit support dans chacune des N zones de révélation, capable de reconnaître ledit biomarqueur présent dans ladite zone,
∘ au moins un moyen de détrompage permettant d'orienter la lecture du dispositif caractérisé en ce qu'il comprend :
   - au moins trois zones permettant la révélation de trois biomarqueurs du microbiote cutané, lesdits biomarqueurs étant des bactéries de chacun des genres *Staphylococcus, Cutibactérium* et *Corynebacterium.*
   - au moins une zone permettant la révélation d'un biomarqueur de cellules de la peau choisi parmi (i) une protéine structurelle, (ii) un biomarqueur de l'inflammation ou (iii) un biomarqueur de l'allergie.

Elle concerne également un kit à usage unique pour la caractérisation de l'état de la peau, un système pour la mise en œuvre d'une recommandation cosmétique personnalisée et une méthode de recommandation cosmétique personnalisée basés sur la caractérisation du complexe peau-microbiote d'un individu et mettant en œuvre le dispositif selon l'invention.

De la même façon que le microbiote intestinal interagit avec le tube digestif dans une symbiose « microbiote-hôte », le microbiote cutané interagit avec la peau aussi en symbiose. De la même façon, il existe autant de type de peaux que de microbiotes, et une atteinte sur un membre de la symbiose se répercute sur l'autre. Ceci est important pour maintenir une peau saine ou pour corriger des imperfections avec des produits de soins cosmétiques. La présente invention permet de faire un relevé biologique rapide et significatif des deux membres de la symbiose grâce à une caractérisation immunochromatographique de marqueurs biologiques accessibles en surface de l'interactome microbiote - peau. Une Intelligence Artificielle permet ensuite de conseiller le meilleur soin adapté d'une part au type de peau et d'autre part compatible avec son microbiote, réduisant les risques d'effets indésirables. De la même façon, en cas d'imperfection de la peau et/ou de déséquilibre du microbiote, la présente invention permet de recommander le meilleur soin correctif, soit garanti compatible avec le microbiote, soit capable de le réguler.

### DESCRIPTION DES FIGURES

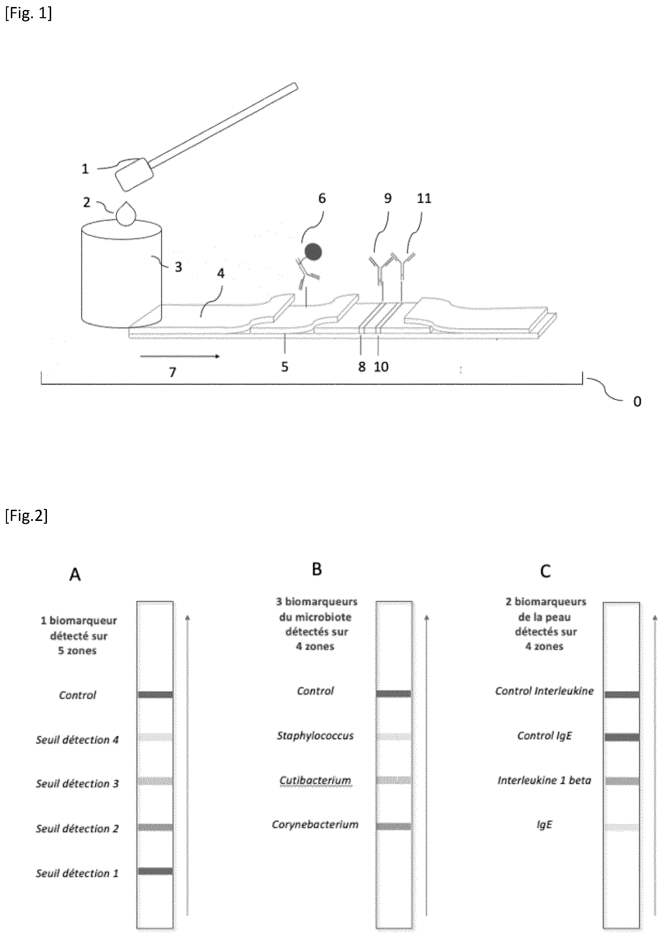

**[****Fig.1]** Figure 1: Représentation d'un mode de réalisation d'un dispositif de révélation immunochromatographique du complexe peau-microbiote selon l'invention comprenant un support (0) sur lequel est aménagé un puits de transfert destiné à recevoir une solution comprenant les constituants du complexe peau-microbiote, ledit puits débouchant sur une pluralité de N zones de révélation. (1) Dispositif de prélèvement (swab, coton-tige...), (2) Solution prélevée sur la peau, (3) Puits de transfert, (4) Zone absorbante de la solution prélevée, (5) Zone avec conjugué de détection (ou anticorps monoclonal de détection), (6) Anticorps monoclonal de détection, (7) Direction du flux de migration, (8) Zone sur laquelle a été déposé un anticorps monoclonal de capture, (9) Anticorps monoclonal (ou fragment d'anticorps, ou nanobody) de capture , (10) Zone de contrôle, (11) Anticorps (ou fragment d'anticorps, ou nanobody) dirigé contre le conjugué.
**[****Fig.2]** Figure 2 : Représentation de 3 modes de réalisation de bandelettes de détection pouvant être mises en œuvre dans un dispositif de révélation immunochromatographique du complexe peau-microbiote selon l'invention. (A) 1 biomarqueur détecté avec plusieurs seuils de détection (par exemple pour un microorganisme : seuil 1 = 10³ cfu ; seuil 2 = 10⁵ cfu ; seuil 3 = 10⁷ cfu ; seuil 4 = 10⁹ cfu ; pour une molécule : seuil 1 = 1 ng ; seuil 2 = 10 ng ; seuil 3 = 100 ng ; seuil 4 = 1000 ng) ; le Control correspond à la détection par un anticorps (ou fragment d'anticorps) (B) 3 biomarqueurs du microbiote détectés sur 4 zones ; le Contrôle correspond à la détection par un anticorps (ou fragment d'anticorps) (C) 2 biomarqueurs de la peau détectés sur 4 zones ; les Controls correspondent à des dépôts des biomarqueurs (validation de la migration du conjugué de détection spécifique de chaque biomarqueur). L'intensité des bandes illustre le fait que les zones de contrôles doivent être à saturation, tandis que l'intensité des autres zones dépend de la quantité d'analyte ayant migré permettant ainsi une analyse semi-quantitative des biomarqueurs.

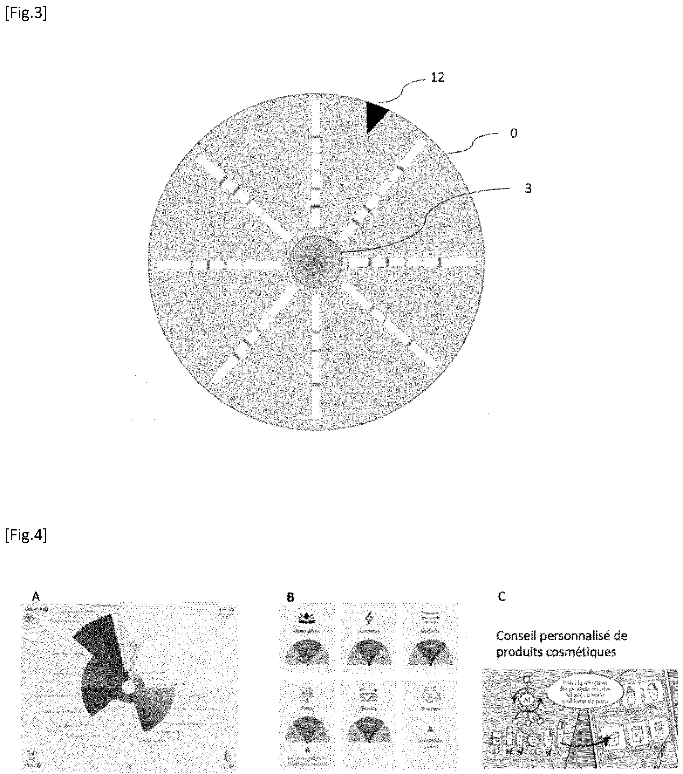

**[****Fig.3]** Figure 3 : Représentation d'un mode de réalisation d'un support (0) pour un dispositif de révélation immunochromatographique du complexe peau-microbiote selon l'invention, sur lequel est aménagé un puits de transfert (central) (3) destiné à recevoir une solution comprenant les constituants du complexe peau-microbiote, ledit puits débouchant sur une pluralité de N zones de révélation disposées sur des bandelettes disposées en rayon autours dudit puits de transfert central (ici 8 bandelettes à titre d'illustration, chacune comprenant 4 ou 5 zones de révélation). Ce support comporte au moins un moyen de détrompage permettant d'orienter la lecture du dispositif (12) matérialisé par un triangle noir qui peut être par exemple une encoche ou une impression et permet d'orienter le sens de lecture du support.
**[****Fig.4]** Figure 4 : Illustration schématique du processus aboutissant à une recommandation cosmétique personnalisée. (A) représentation de l'analyse semi-quantitative du profil du microbiote, le même type d'analyse étant fait pour un ou plusieurs biomarqueurs de la peau (B) présentation du résultat de l'analyse combinatoire des biomarqueurs permettant d'établir la caractérisation du complexe peau-microbiote (C) recommandation de produits cosmétiques adaptés en fonction d'une base de données de produits et mise en relation avec l'état du complexe peau-microbiote.

### DESCRIPTION DETAILLEE DE L'INVENTION

Un premier objet de l'invention concerne un dispositif de révélation immunochromatographique du complexe peau-microbiote comprenant un support sur lequel est aménagé un puits de transfert destiné à recevoir une solution comprenant les constituants du complexe peau-microbiote, ledit puits débouchant sur une pluralité de N zones de révélation portant chacune (i) un réactif de détection immunologique et (ii) un réactif de capture immunologique, et ledit complexe peau-microbiote étant constitué par des cellules de la peau et des microorganismes et autres éléments constitutifs du microbiote cutané, ledit dispositif comprenant :
∘ un nombre de zones de révélation N, N étant égal ou supérieur à 5
∘ au moins un réactif de détection immunologique spécifique d'un biomarqueur de cellules de la peau et au moins un réactif de détection immunologique spécifique d'un biomarqueur du microbiote cutané
∘ lesdits réactifs de détection immunologique comprennent chacun un anticorps spécifique d'un biomarqueur de cellules de la peau ou du microbiote cutané, conjugué à un système de révélation colorimétrique
∘ lesdits réactifs de capture comprennent chacun un anticorps, fixé audit support dans chacune des N zones de révélation, capable de reconnaître ledit biomarqueur présent dans ladite zone,
∘ au moins un moyen de détrompage permettant d'orienter la lecture du dispositif caractérisé en ce qu'il comprend :
   - trois zones permettant la révélation de trois biomarqueurs du microbiote cutané, lesdits biomarqueurs étant trois bactéries des genres *Staphylococcus, Cutibacterium* et *Corynebacterium* respectivement.
   - une zone permettant la révélation d'un biomarqueur de cellules de la peau choisi parmi (i) une protéine structurelle, (ii) un biomarqueur de l'inflammation ou (iii) un biomarqueur de l'allergie.

Le « complexe peau-microbiote » au sens de l'invention, est constitué de deux types cellulaires : cellules de la peau d'origine humaine et microorganismes ; ces deux types cellulaires participent à l'état de la peau en interagissant entre eux. Des phénomènes de symbiose sont observés. Ainsi, en prélevant simultanément les cellules humaines et le microbiote présent à la surface de la peau, on dispose d'un échantillon représentatif d'un état de la peau. Ce complexe contient également d'autres éléments constitutifs du complexe peau-microbiote qui sont sécrétés par les deux types de cellules. D'une part des molécules sécrétées par les cellules de la peau qu'elles soient épidermiques ou immunitaires, mais aussi les sécrétions des microorganismes constitutifs du microbiote.

Il s'agit ici d'établir une caractérisation holistique de l'état de la peau reposant sur la révélation de biomarqueurs représentatifs de l'état physiologique de la peau et de la constitution du microbiote pour permettre leur analyse combinatoire. Le nombre de zones de révélation N correspond au nombre de biomarqueurs testés. N est supérieur à 5 de sorte à pouvoir tester un nombre suffisant de biomarqueurs permettant l'obtention d'un résultat à valeur ajoutée résultant de l'analyse combinatoire des différents biomarqueurs. Le dispositif comprend également au moins une zone témoin ou contrôle, non comprise dans les « au moins 5 zones ».

Les « au moins 5 zones » du dispositif permettent de révéler au moins un biomarqueur de cellules de la peau choisi parmi (i) une protéine structurelle, (ii) un biomarqueur de l'inflammation ou (iii) un biomarqueur de l'allergie et au moins trois biomarqueurs du microbiote cutané, ces trois biomarqueurs étant des bactéries appartenant aux genres *Staphylococcus, Cutibacterium* et *Corynebacterium.* Des modes de réalisation préférés sont décrits ci-après.

Dans des modes de réalisations particuliers de l'invention, au moins 8, 10, 15, 20, 25, 30 types de biomarqueurs différents sont testés simultanément.

Le dispositif permet de révéler l'absence ou la présence des biomarqueurs considérés mais également de les quantifier de manière absolue ou relative, en fonction de la méthode choisie.

### Le microbiote

Le microbiote cutané constitue le premier élément du complexe symbiotique révélé par le dispositif selon l'invention. Il comprend un ensemble de microorganismes parmi lesquels des bactéries, des levures, des champignons, et des acariens.

Afin de fournir une information relative à l'état du microbiote cutané, le dispositif selon l'invention permet au minimum de révéler la présence de bactéries des *genres Staphylococcus, Cutibacterium* et *Corynebacterium.*

De manière préférée, l'espèce de *Staphylococcus* recherchée sera choisie parmi *Staphylococcus epidermidis* et *Staphylococcus aureus.* Ces biomarqueurs pourront être révélés soit via un anticorps capable de reconnaître au moins ces deux espèces parmi le genre *Staphylococcus* (anticorps spécifique du genre), soit via deux anticorps spécifiques des espèces *Staphylococcus epidermidis* et *Staphylococcus aureus* respectivement.

De manière préférée, l'espèce de *Cutibacterium* recherchée sera choisie parmi *Cutibacterium acnes* et *Cutibacterium granulosum.* Ces biomarqueurs pourront être révélés soit via un anticorps capable de reconnaître au moins ces deux espèces parmi le genre *Cutibacterium* (anticorps spécifique du genre), soit via deux anticorps spécifiques des espèces *Cutibacterium acnes* et *Cutibacterium granulosum* respectivement.

De manière préférée, l'espèce de *Corynebacterium* recherchée sera choisie parmi *Corynebacterium xerosis* et *Corynebacterium kroppenstedtii.* Ces biomarqueurs pourront être révélés soit via un anticorps capable de reconnaître au moins ces deux espèces parmi le genre *Corynebacterium* (anticorps spécifique du genre), soit via deux anticorps spécifiques des espèces *Corynebacterium xerosis* et *Corynebacterium kroppenstedtii* respectivement (anticorps spécifique de l'espèce).

Dans le cadre de la présente invention, le microbiote cutané est analysé en révélant des épitopes exposés à la surface du microbiote. Dans un mode de réalisation particulier de l'invention, l'épitope exposé à la surface du microbiote est associé à une protéine présente à la surface d'un microorganisme constitutif du microbiote. Il peut en particulier s'agir d'un épitope associé à un comportement de type biofilm du microbiote.

Les zones de révélation permettent de révéler des « biomarqueurs additionnels du microbiote cutané », c'est à dire d'autres biomarqueurs que ceux qui permettent de révéler la présence de bactéries appartenant aux genres *Staphylococcus, Cutibacterium* ou *Corynebacterium* ou aux espèces associées citées précédemment. Ces autres biomarqueurs peuvent permettre de révéler la présence d'un microorganisme choisi parmi les bactéries, levures, champignons ou acariens.

De manière préférée, les microorganismes constitutifs du microbiote qui seront recherchés pour établir un diagnostic de l'état de la peau dans le cadre de la présente invention sont choisis parmi les bactéries suivantes (Byrd et al., 2018) :
*Staphylococcus epidermidis, Staphylococcus aureus, Staphylococcus lugdunensis, Staphylococcus hominis (H, G), Streptococcus mitis (H, G), Streptococcus oralis (S), Streptococcus pseudopneumoniae (S), Streptococcus sanguinis (S), Staphylococcus capitis,*
*Cutibacterium acnes, Corynebacterium simulans, Corynebacterium fastidiosum (H), Corynebacterium afermentans (H), Corynebacterium xerosis, Corynebacterium aurimucosum (G), Corynebacterium kroppenstedtii (G), Corynebacterium amycolatum (G), Corynebacterium tuberculostearicum,*
*Veillonella parvula (S),*
*Micrococcus luteus (S, H),*
*Enhydrobacter aerosaccus (H),*
*Ou bactéries des genres Dermacoccus, Actinomyces, Bacteroides, Alistipes, Prevotella, Porphyromonas, Sphingobacterium, Lactobacillus, Aerococcus, Oscillospira, Ruminococcus.*

Les autres microorganismes constitutifs du microbiote cutané pouvant être recherchés pour établir un diagnostic selon l'invention sont :
- des levures telles que *Malassezia Globosa, Malassezia restricta, Malassezia furfur, Malassezia sympodialis, Candida parapsilosis...*
- des champignons tels que *Aspergillus tubingensis, Zymoseptoria tritici, Tilletia walkeri, Tilleria Dermacoccus, Epidermophyton floccosum, ,* ....
- des acariens tel que le *Demodex folliculorum, Demodex brevis.*

Ainsi, le dispositif selon l'invention permet d'analyser la présence de microorganismes, éventuellement de caractériser leur organisation sous la forme biofilm, mais également d'analyser la présence au sein du complexe d'autres éléments dont la présence fournie une information intéressante sur l'état de la peau. Dès lors que ces autres éléments sont immunogènes, il est possible de disposer au sein du dispositif, des anticorps capables de révéler leur présence.

Par « épitopes exposés à la surface du microbiote », on entend les épitopes exposés à la surface des microorganismes constitutifs du microbiote via les molécules constitutives de la membrane desdits microorganismes tels que des protéines, des acides gras complexes et des polysaccharides, mais aussi les épitopes formés par les molécules sécrétées comme les exopolysaccharides constitutifs de la matrice du biofilm.

Les cellules de la peau récoltées en surface sont des cellules de *stratum corneum* ou en fonction de l'intensité du prélèvement (abrasion) ou de l'état de la peau (mise à nue après gommage ou exfoliation intense ou peeling), des cellules du *stratum lucida* sous-jacentes ou même du *stratum granulosum* encore plus enfouies en temps normal.

Par « protéines présentes à la surface des microorganismes », on entend les protéines exposées à la membrane : protéines sécrétées et protéines structurelles synthétisées par les cellules qui transitent ou qui sont accrochées à la membrane cellulaire telles que pour les protéines sécrétées. A titre d'illustration, *Staphylococcus aureus* produit la protéine Leucocidine de Pantone-Valentine, et une matrice extracellulaire « polysaccharidique intercellular adhesin » (PIA), *Cutibacterium acnes* produit des porphyrines, *Pseudomonas* une matrice à base d'alginates, Escherichia coli une matrice à base d'acide colanique, *Cutibacterium* une matrice à base de β-1,6-linked N-acetylglucosamine (PNAG) ; pour les protéines structurelles ou d'adhésions à la surface de la peau : pour les bactéries gram positive (simple membrane) : des adhésines de la famille Bap, des pili de type 4 (important pour l'installation précoce du biofilm), des SAATs (Self-associating autotransporters, promoteur de l'agrégation entre bactéries porteuses de SAATs), des intimines/invasines... ; plus spécifiquement pour *Cutibacterium acnes,* des protéines présentes à la surface de la membrane bactérienne, comme des « CAMP factors » (Christie-Atkins-Munch-Petersen factors), des sialidases, des dermatan-sulphate adhesines, des endoglycocéramidases, des chaperonines GroEL, un « SH3 domain-containing lipoprotein », une protéine de type pili/fimbriae Flp, et surtout une protéine DsA1 ; plus spécifiquement pour le genre bactérien Staphylococcus, des MSCRAMMs (molecular surface components recognizing adhesive molecules of the matrix) comme les protéines de la famille Clf-Sdr, avec la Bbp (bone sialoprotein-binding protein), la FnBPs (fibronectin-binding proteins), et la CNA (collagen adhesion), récemment a été identifiée pour *S*. *epidermidis* la protéine SesJ (Arora et al., 2020), tandis que pour *S. aureus,* c'est l'aureusimine (phevalin) qui semble être un marqueur du phénotype biofilm ; alors que pour les bactéries gram négatives (double membrane - LPS) : des adhésines de la famille Bap exhibant un domaine C-terminal LPXTG, des IMPs (Inner-membrane proteins), des pili de type 3 et 4...

Ainsi, les protéines exposées à la membrane des microorganismes constitutifs du microbiote sont choisies parmi la Leucocidine de Pantone-Valentine, les porphyrines, les alginates, β-1,6-linked N-acetylglucosamine (PNAG), les adhésines de la famille Bap, des pili de type 4, des Self-associating autotransporters, les intimines/invasine, CAMP factors, les sialidases, les dermatan-sulphate adhesines, les endoglycocéramidases, les chaperonines GroEL, les protéines de type SH3 domain-containing lipoprotein, la protéine de type pili/fimbriae Flp, la protéine DsA1 ; les protéines de la famille Clf-Sdr, telle que la Bbp, la FnBPs et la CNA, la protéine SesJ, l'aureusimine, les adhésines de la famille Bap exhibant un domaine C-terminal LPXTG, les IMPs, les pili de type 3 et 4.

Les acides gras complexes exposés à la membrane des microorganismes constitutifs du microbiote sont choisis , chez les bactéries gram positives, parmi les acides teichoïques ou même lipotéichoïques, qui peuvent contenir de longues chaînes de ribitol phosphate ou de glycérol-3-phosphate ; tandis que chez les bactéries gram négatives, ils sont choisis parmi les lipopolysaccharides qui sont des glycolipides comprenant une région lipidique appelée lipide A, le plus souvent faite d'un disaccharide de glucosamines phosphorylées et portant des acides gras en liaison ester ou amide.

Les sucres exposés à la membrane des microorganismes constitutifs du microbiote sont choisis parmi les glycopolymères (par exemple à base de rhamnanes), et les peptidoglycanes.

Dans un mode de réalisation préféré de l'invention, au moins un des biomarqueurs spécifiques du microbiote cutané correspond à un biomarqueur associé à un comportement de type biofilm du microbiote. Les bactéries et autres microorganismes sous forme de biofilm se définissent par opposition aux bactéries et autres microorganismes planctoniques, qui sont mobiles et non adhérents. Le biomarqueur spécifique du biofilm peut correspondre à une protéine présente à la surface des membranes des microorganismes ou à un autre élément du microbiote.

Lorsque l'on souhaite rechercher la présence d'une bactérie ou d'un autre microorganisme sous forme de biofilm, les biomarqueurs sont choisis parmi les protéines d'adhésion telles que fimbriae, curli, pili..., caractéristiques de la présence en biofilm du microbiote installé naturellement sur la peau.

### La peau

L'état de la peau constitue le second élément du complexe symbiotique révélé par le dispositif selon l'invention. L'état de la peau est analysé grâce à au moins un biomarqueur de cellules de la peau choisi parmi (i) une protéine structurelle, (ii) un biomarqueur de l'inflammation ou (iii) un biomarqueur de l'allergie.

Les protéines structurelles sont ciblées lorsque l'on souhaite révéler la présence de cellules cutanées et/ou évaluer la qualité de la peau. L'utilisation de biomarqueurs de type protéines structurelles peut également servir à quantifier le nombre de cellules de la peau présentes dans l'échantillon prélevé ou à normaliser le niveau des biomarqueurs. Parmi les protéines structurelles d'intérêt, on peut citer la kératine, la filaggrine, la loricrine...

Les biomarqueurs de l'inflammation sont choisis parmi la Protéine C-Réactive (en anglais C-Reactive Protein, CRP), les interleukines IL-1beta, IL-4, IL-6, IL-8, IL-11, IL-12, le Facteur Nécrosant de Tumeur (en anglais Tumor-Necrosis-Factor-alpha, TNF-alpha), l'Interféron-gamma (IFN-gamma), le facteur Stimulant les Colonies de Granulocytes et de Macrophages (en anglais Granulocytes Macrophages Colony-Stimulating Factor, GM-CSF), le Facteur de Croissance Transformant-beta (en anglais Transforming Growth Factor-beta, TGF-beta).

Les biomarqueurs de l'allergie sont choisis parmi les immunoglobulines, IgE, IgA et IgG, de préférence l'IgE. En effet, le niveau sérique des IgE est élevé dans les cas d'eczémas ou de dermatites atopiques.

Dans un mode de réalisation préféré de l'invention, le « au moins un » biomarqueur des cellules de la peau qui est recherché est l'IL-1béta. Pour les deux autres types de biomarqueurs de cellules de la peau, la protéine structurelle est de préférence la kératine, et le biomarqueur de l'allergie est de préférence l'IgE.

Dans un mode de réalisation préféré de l'invention, le dispositif comprend des zones permettant de révéler spécifiquement la présence des bactéries des genres *Staphylococcus, Cutibacterium* et *Corynebacterium* et de lL-1béta. Dans un mode de réalisation plus particulier, le dispositif comprend en outre une zone permettant de révéler la présence de l'IgE. Dans un mode de réalisation encore plus particulier, il comprend en outre une zone permettant de révéler la présence de la kératine.

### Le dispositif en tant que support des biomarqueurs

Le dispositif de révélation immunochromatographique à usage unique est conditionné de manière stérile et déshydraté. Ce module se présente sous la forme d'un support, typiquement en matière plastique, présentant un puit pour le déversement de la solution de solubilisation contenant la matière biologique récoltée, débouchant sur une série de canaux (par exemple des bandelettes de nitrocellulose ou bandelettes de flux latéral) contenant une pluralité de zones de reconnaissance et révélation spécifiques d'un biomarqueur d'intérêt. Ces canaux peuvent par exemple recevoir des bandelettes de nitrocellulose portant des anticorps, dans lesquels la solution de solubilisation contenant la matière biologique récoltée migre par action capillaire sur la bandelette.

Dans un mode de réalisation préféré, le dispositif selon l'invention est un dispositif immunochromatographique par flux latéral.

Les anticorps de détection peuvent être conjugués soit à une particule d'or de taille nanométrique (ne permet qu'une couleur de marquage en rouge par bandelette, mais différentes lignes de capture), soit à une particule colorée (permet de multiplier le nombre de détection par bandelette), ou par exemple le latex (couleur bleue).

Plusieurs configurations des zones de révélation sont possibles en fonction du mode de révélation des biomarqueurs.

La zone de révélation peut être aménagée sur une bandelette de nitrocellulose avec une seule concentration uniforme d'anticorps de capture sur toute la largeur de la bandelette(zone homogène) pour former un trait révélé par interaction avec l'analyte (devenu visible grâce à l'anticorps-conjugué de détection). Sur une bandelette peuvent être combinées plusieurs zones de détection d'analytes différents, chaque analyte étant détecté avec sa concentration seuil d'anticorps de capture (Figure 2). Dans une autre disposition, cette zone de révélation peut également être aménagée avec au moins deux concentrations différentes d'anticorps de capture. Dans des modes de réalisation alternatifs, la zone comprend 2, 3 ou plus concentrations différentes d'anticorps de capture.

De plus, il est possible de normaliser les seuils de détection des différents biomarqueurs afin de pouvoir établir des quantifications relatives entre les différents biomarqueurs. Cette normalisation peut se faire soit par rapport à un marqueur commun donné servant de référence (par exemple la kératine), soit en établissant la quantification relative de l'ensemble des marqueurs entre eux (par exemple pour ramener à un total cumulé de 100, chaque marqueur obtenant une valeur propre au pro-rata).

Le dispositif doit comprendre un moyen de détrompage, repère nécessaire pour orienter correctement le dispositif lors de la prise de vue de signaux (les traits visibles sur les bandelettes) révélant les biomarqueurs. Ce moyen de détrompage se situe directement sur le dispositif, sur la face de lecture. Il peut se matérialiser sous une forme graphique tel un dessin, ou physique tel une encoche, un trou, ou tout autre système de repérage. Il peut être constitué d'un, deux ou plus éléments de repérage.

Le dispositif est généralement constitué d'un disque, support des zones de révélation, inclus dans une cassette.

Les figures 1 à 3 illustrent différents modes de réalisation de dispositif selon l'invention.

Un second objet de l'invention concerne un kit à usage unique pour la caractérisation de l'état de la peau comprenant :
- Un dispositif de prélèvement du complexe peau-microbiote comprenant un moyen de prélèvement dudit complexe ainsi qu'un réceptacle contenant une solution de solubilisation ;
- Un dispositif de révélation immunochromatographique du complexe peau-microbiote tel que décrit précédemment.

Le moyen de prélèvement peut par exemple être constitué d'un patch, d'une raclette, d'un écouvillon imbibé d'une solution tampon ou d'une éponge imbibée d'une solution tampon d'un tissu de tulle ou de gaze imbibé d'une solution tampon, ou tout autre moyen adapté permettant une action physique d'exfoliation, d'imbibition, d'adsorption.

Le taux de microorganismes, notamment de bactéries, par centimètre carré de peau varie fortement selon les zones de peau, notamment en fonction de la richesse en sébum. Ainsi pour disposer d'un échantillonnage représentatif, il est nécessaire de disposer d'un moyen de prélèvement adapté au type de peau et à la zone prélevée de façon à disposer de suffisamment de matière biologique pour réaliser l'analyse des biomarqueurs de cellules de la peau et du microbiote. La matière biologique prélevée comprend des éléments constitutifs de la partie exposée de la peau (cellules de *stratum corneum*, et/ou *lucidum*, et/ou *granulosum*) et du microbiote (bactéries, levures, champignons, et même acariens comme le demodex).

De manière générale, on considèrera que le prélèvement doit permettre de récolter un nombre suffisant de bactéries pour obtenir une concentration d'au moins 10³ cfu/mL de bactéries par mise en contact avec la solution de solubilisation destinée à recevoir la matière biologique récoltée. De préférence, pour une détection optimale, la concentration de la solution contenant le microbiote récolté sera de 10⁴ cfu/mL de bactéries. Ces concentrations sont toutefois données à titre indicatif et dépendent de la sensibilité de détection de la méthode de révélation.

Lorsque le prélèvement s'effectue via un patch, ce dernier aura une surface d'interaction (surface de collecte) avec la peau d'au moins 5 cm², voire de 10 cm², 12 cm² ou plus, en fonction de l'abondance du microbiote cutané et de l'efficacité du prélèvement. Le moyen de prélèvement (ou de récolte) est complété par un réceptacle (de type tube d'extraction ou équivalent) prérempli et conditionné sous un emballage stérile, contenant un tampon de solubilisation (typiquement entre 1 mL et 5mL), par exemple un tampon de lyse/migration permettant de tamponner le pH de l'échantillon, de minimiser les liaisons non spécifiques, de neutraliser les interférences et de contrôler la vitesse d'écoulement grâce à l'utilisation de divers sels, tensioactifs, détergents, agents stabilisants ou réactifs de blocage (exemple de composition : PBS 1X avec 1% de TweenR20). Ce réceptacle présente un volume adapté à recevoir directement la surface de collecte du dispositif de prélèvement de façon à mettre en solution ce qui aura été prélevé à la surface de la peau.

Le kit à usage unique ne nécessitant que des manipulations simples réalisables immédiatement après le prélèvement sans aucune connaissance biologique et permettant de caractériser un prélèvement par rapport à un grand nombre de biomarqueurs de référence, pour déterminer des combinatoires représentatives d'une grande variété de complexes peau/microbiote.

Un troisième objet de l'invention concerne un système pour la mise en œuvre d'une recommandation cosmétique personnalisée basé sur la caractérisation du complexe peau-microbiote comprenant :
- Un dispositif de prélèvement du complexe peau-microbiote comprenant un moyen de prélèvement dudit complexe ainsi qu'un tube d'extraction contenant une solution de solubilisation ;
- Un dispositif de révélation immunochromatographique du complexe peau-microbiote tel que décrit précédemment, dans lequel lesdites N zones de révélation constituent des zones aptes à fournir un signal lorsqu'un biomarqueur est détecté ;
- Un calculateur exécutant un traitement d'analyse d'image, l'image étant constituée par l'ensemble des signaux desdites N zones révélées grâce audit dispositif de révélation immunochromatographique, pour déterminer la nature des biomarqueurs ayant été reconnus dans lesdites N zones de révélation et catégoriser l'état de la peau en fonction de la combinaison de biomarqueurs identifiée.

Le calculateur réalise une acquisition d'une image des différentes zones de révélation après un temps de réaction donné, et transmet cette image sous une forme numérique à un centre de traitement procédant à une analyse automatique pour caractériser le type de biomarqueurs ayant réagi avec les anticorps présents dans les zones de révélation du dispositif de révélation.

L'acquisition d'images peut se faire en une seule prise de vue de sorte à réaliser une analyse ponctuelle. Ce mode d'analyse est particulièrement adapté à l'utilisation d'un dispositif pour lequel les seuils de détection des anticorps est normalisé, ou bien les zones de révélation comportent au moins deux concentrations différentes d'anticorps.

L'acquisition d'images peut également se faire en plusieurs prise de vues successives afin de réaliser une analyse dynamique. Cette méthode permet de suivre l'apparition des signaux et de réaliser des quantifications relatives des biomarqueurs. Elle peut être mise en œuvre à partir d'un dispositif pour lequel le seuil de détection des anticorps est normalisé ou non. Une disposition peut prévoir par exemple un suivi en temps réel de l'évolution de l'intensité des signaux (intensité des traits de chaque zone sur les bandelettes) pour déclencher une première prise de vue lorsque le biomarqueur de référence atteint une intensité correspondant au niveau de saturation (par exemple après 5 minutes de migration) et une deuxième prise de vue lorsque le biomarqueur présentant le signal le plus faible atteint une intensité correspondant au seuil minimum de détection (par exemple 10 minutes), ou lorsque le processus de migration est estimé parvenu à son terme (par exemple 15 minutes).

La prise de vue peut être réalisée par Smartphone ou équivalent, associé à un logiciel d'analyse d'image pour la traiter en exploitant les données accumulées (cette analyse peut faire intervenir un logiciel entrainé basé sur des processus d'apprentissage de type IA comprenant à la fois les données renseignées par l'utilisateur - âge, poids, taille, consommation de tabac, d'alcool, susceptibilité au sucre (diabète), séances en cabines UV...). Il est ensuite possible de scanner les codes-barres des produits utilisés pour la routine cosmétique, pour une mise en relation avec les informations relatives aux biomarqueurs de la peau et aux biomarqueurs du microbiote, afin d'établir une recommandation de produits (nutraceutiques, cosmétiques...) les plus adaptés à l'état de la peau et au microbiote.

Ces trois moyens forment un ensemble indissociable : l'analyse des biomarqueurs via le dispositif de révélation immunochromatographique est possible seulement parce que le dispositif de prélèvement permet le recueil d'un volume suffisant de matière biologique (cellule de la peau et microbiote).

L'utilisation de ce kit pour une analyse complexe en raison du grand nombre de biomarqueurs analysés à la fois sur les cellules de la peau et sur le microbiote cutané, et d'une combinatoire correspondant à un grand nombre d'arrangements nécessaire pour catégoriser la matière biologique, ne permettant pas de tirer des enseignements par une lecture simple comme pour un test Covid ou un test de grossesse, n'est possible qu'en raison de la simplicité de l'acquisition d'une image et de sa transmission sous forme numérique pour un traitement sur un calculateur mutualisant les traitements des kits pour un grand nombre d'utilisateurs.

Les canaux présentant les zones de détection définissent un plan fermé par une fenêtre transparente. L'utilisateur utilise cette fenêtre pour prendre une photographie après écoulement du temps de réaction, à l'aide par exemple d'un téléphone intelligent exécutant une application dédiée commandant notamment :
a) l'acquisition d'une image,
b) la vérification locale, sur le calculateur du téléphone intelligent, de la conformité de l'image acquise par rapport à des mires prévues sur le dispositif d'imagerie par exemple
c) la saisie d'information additionnelle
d) l'horodatage et la géolocalisation éventuelle du téléphone intelligent
e) la transmission de l'image et des informations associées à un serveur distant

### Exploitation des images

Les images reçues sur le serveur font l'objet ensuite d'un traitement automatique de reconnaissance des zones de détection ayant réagi pour codifier la combinaison de biomarqueurs présente dans le complexe peau-microbiote analysé, et catégoriser la peau par un traitement de cette combinaison, notamment par apprentissage supervisé à partir d'une base de référence portant sur des recueils sur un panel de personnes ayant fait l'objet d'une caractérisation par un expert.

Un quatrième objet de l'invention concerne une méthode de recommandation cosmétique personnalisée comprenant les étapes suivantes :
- Prélèvement d'un échantillon de matière biologique à la surface de la peau à l'aide d'un moyen de prélèvement
- Mise en suspension des cellules de la peau et des microorganismes constitutifs de la matière biologique prélevée dans une solution de solubilisation
- Déversement de ladite solution de solubilisation dans le puits de transfert d'un dispositif de révélation immunologique tel que défini précédemment
- Incubation entre 1 et 10 min afin de laisser réagir les cellules et les microorganismes avec les réactifs sur chacune des N zones dudit dispositif de révélation immunologique
- Traitement de l'image constituée par l'ensemble des signaux révélés sur chacune des N zones via un calculateur permettant de catégoriser la peau par un traitement de cette combinaison de signaux, notamment par apprentissage supervisé à partir d'une base de référence
- Recommandation d'un ou de plusieurs produits cosmétiques adaptés à la peau dudit individu en fonction de la combinaison de biomarqueurs révélée.

La recommandation est établie sur la base d'un arbre décisionnel prenant en compte les informations relatives à un état de peau irritée et/ou infectée, qui se traduit par exemple par les critères suivants :
1 - Information sur le niveau d'irritation ou d'inflammation de la peau telle que la présence d'une dermatite atopique, d'un eczéma, d'une rosacée... grâce aux marqueurs biologiques « peau » suivants :
   - Marqueurs biologiques : kératine (témoin qualité du prélèvement), filaggrine..., IgE, CRP, IL-6... ;
   - CRP : peut varier en fonction cycle menstruel, marqueur infection ;
   - Si marqueur kératine faible = gommage ou exfoliation récent et trop agressif, peeling ;
2 - Information sur la composition du microbiote cutané (niveau de colonisation/contamination de la peau).

Le microbiote est considéré « normal » si la colonisation est de type « commensale », le microbiote étant constitué de microorganismes attendus.

Une contamination ou une infection sera détectée si le marqueur biologique du microorganisme cible est élevé, par exemple :
- Présence d'acné si C. *acnes* est élevé
- Présence d'une dermatite atopique (eczéma) infectée si le marqueur *S. aureus* est élevé
- Présence d'une rosacée si le marqueur *S. epidermidis* ou le marqueur *Demodex* est élevé
- Risque de pellicules sur le cuir chevelu si le marqueur *Malassezia* est élevé.

En ce qui concerne le microbiote cutané, l'analyse renseigne sur l'équilibre entre différentes populations de microorganismes et permet de détecter l'existence de dysbiose.

La figure 4 illustre les étapes de cette méthode de recommandation.

### Exemple non limitatif d'un mode de réalisation de l'invention

La présente invention sera mieux comprise à la lecture de la description qui suit, concernant un exemple non limitatif de réalisation où :
Une personne constate l'apparition de boutons sur son visage, elle estime sa peau plutôt grasse, elle utilise une routine de soin lui convenant (conseils maternels, d'amies, d'esthéticiennes etc), elle a entendu parler du microbiote intestinal et de son impact sur la santé (stimulation du système immunitaire, protection contre des germes pathogènes pouvant irriter ou même agresser le tube digestif etc), il existe des tests mais ce n'est pas pratique, il faut commander un kit de prélèvement sur un site internet, c'est assez cher (250 à 500€), réaliser le prélèvement à domicile et ensuite le renvoyer à un laboratoire qui réalise une analyse « métagénomique », et ensuite attendre plusieurs semaines pour obtenir un résultat et des conseils. Il faut être très motivé et patient.

Avec le dispositif selon l'invention, il suffit de se rendre dans une boutique cosmétique, ou une pharmacie (cosmétique et hygiène en OTC), ou de le commander via une application smartphone. Même s'il est recommandé de réaliser le test le matin après s'être démaquillé et rincé la peau du visage la veille, on peut aussi procéder au test de façon extemporanée (avec le risque de n'identifier que les 3 ou 4 principaux microorganismes du microbiote, mais cela peut être suffisant pour confirmer une suspicion d'acné, par exemple). Le dispositif se présente la forme d'un kit avec un dispositif simple de prélèvement sur la peau, on applique un petit dispositif spongieux, absorbant, légèrement abrasif imbibé d'une solution optimisant le prélèvement puis on insère ce dispositif dans un tube contenant une solution dite « de resuspension » (ou de « solubilisation »).

La solution s'écoule dans le dispositif ICFL constitué d'une feuille de nitrocellulose absorbante (nitrocellulose = NC) qui permet de créer un flux de migration, avec une zone d'entrée au-dessus de laquelle est disposée une autre feuille de NC où un « conjugué de détection » aura été déposé. Ce conjugué de détection est en général un anticorps monoclonal dirigé contre un antigène cible, l'analyte recherché, c'est-à-dire une protéine caractéristique de l'état de la peau (protéine de structure -kératine, filaggrine...-, de l'inflammation - CRP, ...- , du système immunitaire - IL6, ...- etc) ou du microbiote (protéines d'adhésion fimbriae, curli, pili..., caractéristiques de la présence en biofilm du microbiote installé naturellement sur la peau, exprimées par les 3 bactéries majeures *Staphylococcus epidermidis, Cutibacterium acnes, Staphylococcus aureus,* et d'autres plus minoritaires, certaines communes à tout type de peau et d'autres spécifiques d'une peau sèche, ou grasse ou humide). Le conjugué de détection est appelé ainsi parce qu'y est fixé un composé (microparticule d'or, microbilles de latex, etc.) qui permettra la visualisation de l'interaction antigène-anticorps.

Après cette première interaction à l'entrée du puits de dépôt, le flux progresse vers une zone de détection sur laquelle a été déposé un anticorps monoclonal de capture aussi dirigé contre les antigènes caractéristiques de l'état de la peau et du microbiote. Chaque anticorps de capture est déposé selon une ligne test dont le résultat indiquera la présence ou l'absence de l'analyte.

Il y a systématiquement, en plus, une ligne de contrôle dont le résultat permet la validation d'une bonne migration et donc la validation du test. Pour qu'il y ait une réaction sur cette zone, l'anticorps fixe sur la membrane au niveau de la ligne de contrôle est dirigé contre le conjugué.

Sur chaque dispositif ICFL sont prévus plusieurs détections d'analytes (nécessitant plusieurs couples « conjugué de détection et anticorps de capture »). Ce qui permet de déterminer un profil de l'état de la peau (saine, normale, sèche, grasse, etc) associé à un profil de microbiote (sain, caractéristique d'une peau sèche, grasse, susceptible à l'acné, à la dermatite etc).

Lecture et analyse sont réalisées par prise d'image avec smartphone, interprétation par IA. Les informations données par les biomarqueurs peuvent de plus être combinées aux renseignements apportés par la personne.

### Exemple de mise en œuvre du dispositif pour une recommandation cosmétique

La présente invention permet ainsi différents types de tests :
i. Test ponctuel en 15 à 30 minutes (possible en boutique, chez soi à domicile en « Home test »), soit en démarche spontanée , soit en réaction à un évènement : utilisation d'un produit cosmétique ou d'hygiène irritant, apparition d'un érythème solaire suite à une exposition prolongée au soleil, ou suite à une séance en cabine UV artificiels, ou encore après un gommage ou une exfoliation ou un peeling trop intense, ou encore diagnostic d'une pathologie de la peau (affections cutanées les plus fréquentes étant l'acné, l'eczéma, le psoriasis, les maladies du cuir chevelu (hors pelade), les mycoses et les maladies des ongles ;
ii. Des tests répétés, successifs permettant un suivi dans le temps (monitoring) de l'état général de la peau en y associant le microbiote résidant (complexe peau-microbiote), ce qui peut conduire à mieux gérer les expositions au soleil, aux UV artificiels, ou espacer ou atténuer les pratiques de gommage, ou d'exfoliation ou de peeling ;
iii. Selon le profil obtenu, la proposition de produits adaptés reliant l'état de la peau à un traitement *ad hoc* (peau sèche = routine d'hydratation, peau grasse = routine exfoliation nettoyante, peau fine et ridée = routine crème de protection et nourrissante...) avec l'information « microbiote » associée : soit compléments « probiotiques » stimulant la peau et/ou le microbiote, soit produits « compatibles » pour éviter un traitement de la peau seule au détriment du microbiote (une campagne de tests desdits produits versus microorganismes du microbiote peut permettre de calculer un score de compatibilité), soit produit de soin pouvant améliorer un syndrome désagréable (sensation de peau sèche, grasse, irritée, rougeur etc) ;
iv. De vérifier la compatibilité des produits utilisés en routine. De faire évoluer la routine en fonction de l'évolution du complexe peau-microbiote avec l'âge (où la peau devient plus fine), le climat (été chaud et humide versus hiver froid et sec), la pollution etc ).

## Revendications

1. Méthode de recommandation cosmétique personnalisée basée sur la caractérisation immunochromatographique, simultanément et immédiatement après le prélèvement, de différents biomarqueurs du complexe peau-microbiote d'un individu comprenant les étapes suivantes :
1) Mise à disposition d'un échantillon de matière biologique prélevée à la surface de la peau et contenant les éléments constitutifs du complexe peau-microbiote, ledit complexe peau-microbiote étant constitué par des cellules de la peau et des microorganismes et autres éléments constitutifs du microbiote cutané,
2) Mise en suspension dudit complexe peau-microbiote dans une solution de solubilisation
3) Déversement de ladite solution de solubilisation dans le puits de transfert d'un dispositif de révélation immunochromatographique, ledit dispositif de révélation immunochromatographique comprenant un support sur lequel est aménagé un puits de transfert destiné à recevoir ladite solution de solubilisation, ledit puits débouchant sur une pluralité de N zones de révélation portant chacune (i) un réactif de détection immunologique et (ii) un réactif de capture immunologique, et, ledit dispositif de révélation immunologique comprenant :
∘ au moins un réactif de détection immunologique spécifique d'un biomarqueur de cellules de la peau et au moins un réactif de détection immunologique spécifique d'un biomarqueur du microbiote cutané
∘ lesdits réactifs de détection immunologique comprennent chacun un anticorps spécifique d'un biomarqueur de cellules de la peau ou du microbiote cutané, conjugué à un système de révélation colorimétrique
∘ lesdits réactifs de capture immunologique comprennent chacun un anticorps, fixé audit support dans chacune des N zones de révélation, capable de reconnaître ledit biomarqueur présent dans ladite zone,
∘ au moins un moyen de détrompage permettant d'orienter la lecture du dispositif
4) Incubation entre 1 et 10 min afin de laisser réagir les éléments du complexe peau-microbiote avec lesdits réactifs sur chacune des N zones dudit dispositif de révélation immunologique
5) Traitement de l'image constituée par l'ensemble des signaux révélés sur chacune des N zones de révélation via un calculateur permettant de catégoriser le complexe peau-microbiote par un traitement de cette combinaison de signaux
6) Recommandation d'un ou de plusieurs produits cosmétiques en fonction de la combinaison de biomarqueurs révélée
**caractérisée en ce que** :
∘ le nombre N de zones de révélation est supérieur ou égal à 5 et comporte a) au moins trois zones permettant la révélation de trois biomarqueurs du microbiote cutané, lesdits biomarqueurs étant des bactéries des genres *Staphylococcus, Cutibacterium* et *Corynebacterium* et b) au moins une zone permettant la révélation d'un biomarqueur de cellules de la peau choisi parmi (i) une protéine structurelle, (ii) un biomarqueur de l'inflammation ou (iii) un biomarqueur de l'allergie.
∘ lesdits produits cosmétiques recommandés sont adaptés, d'une part au type de peau dudit individu et sont, d'autre part, compatibles avec son microbiote.

2. Méthode selon la revendication 1 dans laquelle ladite recommandation d'un ou plusieurs produits cosmétiques est établie sur la base d'un arbre décisionnel basé sur les critères suivants :
1) l'état physiologique de la peau établi grâce à l'analyse des signaux relatifs aux biomarqueurs de cellules de la peau
2) la composition du microbiote établie grâce à l'analyse des signaux relatifs aux biomarqueurs du microbiote, y compris celle de l'équilibre entre différentes populations de microorganismes

3. Méthode selon la revendication 1 ou 2 **caractérisée en ce que** l'analyse des biomarqueurs est une analyse semi-quantitative.

4. Méthode selon l'une des revendications précédentes dans laquelle ladite bactérie du genre *Staphylococcus* est choisie parmi *Staphylococcus epidermidis* et *Staphylococcus aureus,* la bactérie du genre *Cutibacterium* est choisie parmi *Cutibacterium acnes* et *Cutibacterium granulosum* et la bactérie du genre *Corynebacterium* est choisie parmi *Corynebacterium xerosis* et *Corynebacterium kroppenstedtii*

5. Méthode selon l'une des revendications précédentes dans laquelle au moins un des biomarqueurs spécifiques du microbiote cutané correspond à un épitope associé à un comportement de type biofilm du microbiote.

6. Méthode selon l'une des revendications précédentes dans laquelle au moins une zone de révélation spécifique d'un biomarqueur additionnel du microbiote cutané est un microorganisme choisi parmi les bactéries, les levures, les champignons et les acariens.

7. Méthode selon l'une des revendications précédentes dans laquelle ledit biomarqueur additionnel du microbiote cutané est choisi parmi les espèces suivantes :
(i) une bactérie choisie parmi *Staphylococcus epidermidis, Staphylococcus aureus, Staphyloccocus hominis, Staphylococcus lugdunensis, Staphylococcus capitis, Cutibacterium acnes, Propionibacterium granulosum, Propionibacterium avidum, Corynebacterium tuberculostearicum, Corynebacterium simulans, Corynebacterium fastidiosum, Corynebacterium afermentans, Corynebacterium xerosis, Corynebacterium aurimucosum), Corynebacterium kroppenstedtii, Corynebacterium amycolatum, Streptococcus mitis, Streptococcus orali), Streptococcus pseudopneumoniae, Streptococcus sanguinis, Veillonella parvula, Micrococcus luteus, Enhydrobacter aerosaccus, Actinomyces*
(ii) une levure choisie parmi *Malassezia globosa, Malassezia restricta, Malassezia furfur* ou *Candida parapsilosis*
(iii) un champignon choisi parmi *Aspergillus tubingensis, Zymoseptoria tritici, Tilletia walkeri, Epidermophyton floccosum, Nannizzia nana,* (iv) un acarien choisi parmi *Demodex folliculorum, Demodex brevis.*

8. Méthode selon l'une des revendications précédentes dans laquelle ledit biomarqueur du microbiote est une protéine exposée à la surface dudit microorganisme choisie parmi la Leucocidine de Pantone-Valentine, les porphyrines, les alginates, β-1,6-linked N-acetylglucosamine (PNAG), les adhésines de la famille Bap, des pili de type 4, des Self-associating autotransporters, les intimines/invasine, les CAMP factors, les sialidases, les dermatan-sulphate adhesines, les endoglycocéramidases, les chaperonines GroEL, les protéines de type SH3 domain-containing lipoprotein, la protéine de type pili/fimbriae Flp, la protéine DsA1 ; les protéines de la famille Clf-Sdr, telle que la Bbp, la FnBPs et la CNA, la protéine SesJ, l'aureusimine, les adhésines de la famille Bap exhibant un domaine C-terminal LPXTG, les IMPs, les pili de type 3 et 4.

9. Méthode selon l'une des revendications précédentes dans laquelle lesdits biomarqueurs des cellules de la peau sont choisis parmi (i) une protéine structurelle choisie parmi la kératine, la filaggrine, la loricrine, (ii) un biomarqueur de l'inflammation choisi parmi la Protéine C-Réactive, les interleukines IL-1beta, IL-4, IL-6, IL-8, IL-11, IL-12, le Facteur Nécrosant de Tumeur, l'Interféron-gamma, le facteur stimulant les colonies de granulocytes et de macrophages, le Facteur de Croissance Transformant-beta et (iii) un biomarqueur de l'allergie choisi parmi les immunoglobulines IgE, IgA et IgG.

10. Méthode selon l'une des revendications précédentes dans laquelle ledit biomarqueur de cellules de la peau est l'interleukine 1-béta.

11. Méthode selon l'une des revendications précédentes **caractérisée en ce qu'**il comprend des zones permettant de révéler spécifiquement la présence des bactéries des genres *Staphylococcus, Cutibacterium* et *Corynebacterium* et de IL-1béta.

12. Méthode selon l'une des revendications précédentes **caractérisée en ce que** ledit dispositif est un dispositif immunochromatographique par flux latéral.

13. Méthode selon l'une des revendications précédentes **caractérisée en ce que** la concentration en bactéries de la solution de solubilisation après mise en contact avec ladite matière biologique prélevée est de l'ordre de 10⁴ cfu/mL.

## Patentansprüche

1. Verfahren für eine personalisierte kosmetische Empfehlung basierend auf der immunchromatographischen Charakterisierung, gleichzeitig und unmittelbar nach der Probeentnahme, verschiedener Biomarker des Haut-Mikrobiota-Komplexes eines Individuums, umfassend die folgenden Schritte:
1) Bereitstellen einer Probe von biologischem Material, die von der Hautoberfläche entnommen wird und die Bestandteile des Haut-Mikrobiota-Komplexes enthält, wobei der Haut-Mikrobiota-Komplex aus Hautzellen und Mikroorganismen und anderen Bestandteilen der kutanen Mikrobiota besteht,
2) Suspendieren des Haut-Mikrobiota-Komplexes in einer Solubilisierungslösung
3) Schütten der Solubilisierungslösung in die Transfersenke einer immunchromatographischen Aufdeckungsvorrichtung, die immunchromatographische Aufdeckungsvorrichtung umfassend einen Träger, auf dem eine Transfersenke angeordnet ist, die dazu bestimmt ist, die Solubilisierungslösung aufzunehmen, wobei die Senke in eine Vielzahl von N Aufdeckungszonen mündet, die jeweils (i) ein immunologisches Nachweisreagenz und (ii) ein immunologisches Einfangreagenz tragen, und die immunologische Aufdeckungsvorrichtung umfassend:
∘ mindestens ein für einen Biomarker von Hautzellen spezifisches immunologisches Nachweisreagenz und mindestens ein für einen Biomarker der kutanen Mikrobiota spezifisches immunologisches Nachweisreagenz
∘ wobei die immunologischen Nachweisreagenzien jeweils einen für einen Biomarker von Hautzellen oder der kutanen Mikrobiota spezifischen Antikörper umfassen, der an ein kolorimetrisches Aufdeckungssystem konjugiert ist
∘ wobei die immunologischen Einfangreagenzien jeweils einen Antikörper umfassen, der an dem Träger in jeder der N Aufdeckungszonen fixiert ist, der in der Lage ist, den in der Zone vorhandenen Biomarker zu erkennen,
∘ mindestens ein Unverwechselbarkeitsmittel, das es ermöglicht, das Ablesen der Vorrichtung auszurichten
4) Inkubieren zwischen 1 und 10 min, um die Elemente des Haut-Mikrobiota-Komplexes mit den Reagenzien auf jeder der N Zonen der immunologischen Aufdeckungsvorrichtung reagieren zu lassen
5) Verarbeiten des Bildes, das aus der Gesamtheit der auf jeder der N Aufdeckungszonen aufgedeckten Signale besteht, mittels eines Rechners, der es ermöglicht, den Haut-Mikrobiota-Komplex durch Verarbeiten dieser Signalkombination zu kategorisieren
6) Empfehlen eines oder mehrerer kosmetischer Mittel in Abhängigkeit von der aufgedeckten Biomarkerkombination
**dadurch gekennzeichnet, dass**:
∘ die Anzahl N der Aufdeckungszonen größer als oder gleich 5 ist und a) mindestens drei Zonen, die die Aufdeckung von drei Biomarkern der kutanen Mikrobiota ermöglichen, wobei die Biomarker Bakterien der Gattungen *Staphylococcus, Cutibacterium* und *Corynebacterium* sind, und b) mindestens eine Zone aufweist, die die Aufdeckung eines Biomarkers von Hautzellen ermöglicht, ausgewählt aus (i) einem Strukturprotein, (ii) einem Entzündungsbiomarker oder (iii) einem Allergiebiomarker.
∘ die empfohlenen kosmetischen Produkte einerseits an den Hauttyp des Individuums angepasst sind und andererseits mit seiner Mikrobiota kompatibel sind.

2. Verfahren nach Anspruch 1, wobei die Empfehlung eines oder mehrerer kosmetischer Produkte auf der Basis eines Entscheidungsbaums erstellt wird, der auf den folgenden Kriterien basiert:
1) dem physiologische Zustand der Haut, der durch die Analyse der Signale in Bezug auf Biomarker von Hautzellen ermittelt wird
2) der Zusammensetzung der Mikrobiota, der durch die Analyse der Signale in Bezug auf Biomarker der Mikrobiota ermittelt wird, einschließlich derjenigen des Gleichgewichts zwischen verschiedenen Populationen von Mikroorganismen

3. Verfahren nach Anspruch 1 oder 2, **dadurch gekennzeichnet, dass** die Analyse der Biomarker eine semiquantitative Analyse ist.

4. Verfahren nach einem der vorstehenden Ansprüche, wobei das Bakterium der Gattung *Staphylococcus* ausgewählt ist aus *Staphylococcus epidermidis* und *Staphylococcus aureus,* das Bakterium der Gattung *Cutibacterium* ausgewählt ist aus *Cutibacterium acnes* und *Cutibacterium granulosum* und das Bakterium der Gattung *Corynebacterium* ausgewählt ist aus *Corynebacterium xerosis und Corynebacterium kroppenstedtii*

5. Verfahren nach einem der vorstehenden Ansprüche, wobei mindestens einer der spezifischen Biomarker der kutanen Mikrobiota einem Epitop entspricht, das mit einem Verhalten des Biofilmtyps der Mikrobiota assoziiert ist.

6. Verfahren nach einem der vorstehenden Ansprüche, wobei mindestens eine für einen zusätzlichen Biomarker der kutanen Mikrobiota spezifische Aufdeckungszone ein Mikroorganismus ist, ausgewählt aus Bakterien, Hefen, Pilzen und Milben.

7. Verfahren nach einem der vorstehenden Ansprüche, wobei der zusätzliche Biomarker der kutanen Mikrobiota aus den folgenden Arten ausgewählt ist:
(i) einem Bakterium, ausgewählt aus *Staphylococcus epidermidis, Staphylococcus aureus, Staphyloccocus hominis, Staphylococcus lugdunensis, Staphylococcus capitis, Cutibacterium acnes, Propionibacterium granulosum, Propionibacterium avidum, Corynebacterium tuberculostearicum, Corynebacterium simulans, Corynebacterium fastidiosum, Corynebacterium afermentans, Corynebacterium xerosis, Corynebacterium aurimucosum), Corynebacterium kroppenstedtii, Corynebacterium amycolatum, Streptococcus mitis, Streptococcus orali), Streptococcus pseudopneumoniae, Streptococcus sanguinis, Veillonella parvula, Micrococcus luteus, Enhydrobacter aerosaccus, Actinomyces*
(ii) einer Hefe, ausgewählt aus *Malassezia globosa, Malassezia restricta, Malassezia furfur* oder *Candida parapsilosis*
(iii) einem Pilz, ausgewählt aus *Aspergillus tubingensis, Zymoseptoria tritici, Tilletia walkeri, Epidermophyton floccosum, Nannizzia nana,* (iv) eine Milbe, ausgewählt aus *Demodex folliculorum, Demodex brevis.*

8. Verfahren nach einem der vorstehenden Ansprüche, wobei der Biomarker der Mikrobiota ein auf der Oberfläche des Mikroorganismus exponiertes Protein ist, ausgewählt aus Panton-Valentine-Leukozidin, Porphyrinen, Alginaten, β-1,6-verknüpftem N-Acetylglucosamin (PNAG), Adhäsinen der Bap-Familie, Typ-4-Pili, selbstassoziierenden Autotransportern, Intiminen/Invasin, CAMP-Faktoren, Sialidasen, Dermatansulfat-Adhäsinen, Endoglykoceramidasen, GroEL-Chaperoninen, Proteinen vom Typ SH3-Domänen-haltiges Lipoprotein, dem Protein vom Typ Flp-Pili/Fimbrien, dem DsA1-Protein; Proteinen der Clf-Sdr-Familie, wie etwa Bbp, FnBPs und CNA, dem SesJ-Protein, Aureusimin, Adhäsinen der Bap-Familie, die eine C-terminale LPXTG-Domäne vorweisen, IMPs, Pili vonTyp 3 und 4.

9. Verfahren nach einem der vorstehenden Ansprüche, wobei die Biomarker der Hautzellen ausgewählt sind aus (i) einem Strukturprotein, ausgewählt aus Keratin, Filaggrin, Loricrin, (ii) einem Entzündungsbiomarker, ausgewählt aus C-reaktivem Protein, den Interleukinen IL-1beta, IL-4, IL-6, IL-8, IL-11, IL-12, Tumornekrosefaktor, Interferon-gamma, Granulozyten-Makrophagen-Koloniestimulierendem Faktor, transformierendem Wachstumsfaktor-beta und (iii) einem Allergiebiomarker, ausgewählt aus den Immunglobulinen IgE, IgA und IgG.

10. Verfahren nach einem der vorstehenden Ansprüche, wobei der Hautzellen-Biomarker Interleukin-1-beta ist.

11. Verfahren nach einem der vorstehenden Ansprüche,
**dadurch gekennzeichnet, dass** es Zonen umfasst, die es ermöglichen, das Vorhandensein von Bakterien der Gattungen *Staphylococcus, Cutibacterium* und *Corynebacterium* und von IL-1beta spezifisch aufzudecken.

12. Verfahren nach einem der vorstehenden Ansprüche,
**dadurch gekennzeichnet, dass** die Vorrichtung eine immunchromatographische Lateral-Flow-Vorrichtung ist.

13. Verfahren nach einem der vorstehenden Ansprüche,
**dadurch gekennzeichnet, dass** die Bakterienkonzentration der Solubilisierungslösung nach dem Inkontaktbringen mit dem entnommenen biologischen Material in der Größenordnung von 10⁴ cfu/mL liegt.

## Claims

1. A personalized cosmetic recommendation method based on immunochromatographically characterizing, simultaneously and immediately after sampling, different biomarkers of the skin-microbiota complex of an individual, comprising the following steps:
1) providing a sample of biological material taken from the surface of the skin and containing the constituent elements of the skin-microbiota complex, the skin-microbiota complex consisting of skin cells and microorganisms and other constituent elements of the skin microbiota,
2) suspending the skin-microbiota complex in a solubilization solution
3) pouring the solubilization solution into the transfer well of an immunochromatographic detection device, the immunochromatographic detection device comprising a support on which a transfer well intended to receive the solubilization solution is arranged, the well leading to a plurality of N detection zones each having (i) an immunological detection reagent and (ii) an immunological capture reagent, and the immunological detection device comprising:
∘ at least one immunological detection reagent specific to a skin cell biomarker, and at least one immunological detection reagent specific to a biomarker of the skin microbiota
∘ the immunological detection reagents each comprise an antibody which is specific to a skin cell biomarker or biomarker of the skin microbiota and is conjugated to a colorimetric detection system
∘ the immunological capture reagents each comprise an antibody which is bound to the support in each of the N detection zones and is capable of recognizing the biomarker present in the zone,
∘ at least one foolproofing means for orienting the reading of the device
4) incubating for between 1 and 10 min in order to allow the elements of the skin-microbiota complex to react with the reagents on each of the N zones of the immunological detection device
5) processing the image consisting of the set of signals detection on each of the N detection zones via a computer for categorizing the skin-microbiota complex by processing this combination of signals
6) recommending one or more cosmetic products on the basis of the combination of biomarkers detected
**characterized in that**:
∘ the number N of detection zones is greater than or equal to 5 and comprises a) at least three zones for detecting three biomarkers of the skin microbiota, the biomarkers being bacteria of the genera *Staphylococcus, Cutibacterium* and *Corynebacterium* and b) at least one zone for detecting a skin cell biomarker selected from (i) a structural protein, (ii) an inflammatory biomarker or (iii) an allergy biomarker.
∘ the recommended cosmetic products are both suited to the skin type of the individual and compatible with the microbiota thereof.

2. The method according to claim 1, wherein the recommendation of one or more cosmetic products is established on the basis of a decision tree based on the following criteria:
1) the physiological state of the skin established by virtue of analyzing signals relating to the skin cell biomarkers
2) the composition of the microbiota established by virtue of analyzing signals relating to the biomarkers of the microbiota, including the balance between different populations of microorganisms

3. The method according to claim 1 or 2, **characterized in that** the biomarker analysis is a semi-quantitative analysis.

4. The method according to any of the preceding claims, wherein the bacterium of the genus *Staphylococcus* is selected from *Staphylococcus epidermidis* and *Staphylococcus aureus,* the bacterium of the genus *Cutibacterium* is selected from *Cutibacterium acnes* and *Cutibacterium granulosum* and the bacterium of the genus *Corynebacterium* is selected from *Corynebacterium xerosis and Corynebacterium kroppenstedtii*

5. The method according to any of the preceding claims, wherein at least one of the biomarkers specific to the skin microbiota corresponds to an epitope associated with biofilm-like behavior of the microbiota.

6. The method according to any of the preceding claims, wherein at least one detection zone specific to an additional biomarker of the skin microbiota is a microorganism selected from bacteria, yeasts, fungi and mites.

7. The method according to any of the preceding claims, wherein the additional biomarker of the skin microbiota is selected from the following species:
(i) a bacterium selected from *Staphylococcus epidermidis, Staphylococcus aureus, Staphyloccocus hominis, Staphylococcus lugdunensis, Staphylococcus capitis, Cutibacterium acnes, Propionibacterium granulosum, Propionibacterium avidum, Corynebacterium tuberculostearicum, Corynebacterium simulans, Corynebacterium fastidiosum, Corynebacterium afermentans, Corynebacterium xerosis, Corynebacterium aurimucosum), Corynebacterium kroppenstedtii, Corynebacterium amycolatum, Streptococcus mitis, Streptococcus orali), Streptococcus pseudopneumoniae, Streptococcus sanguinis, Veillonella parvula, Micrococcus luteus, Enhydrobacter aerosaccus, Actinomyces*
(ii) a yeast selected from *Malassezia globosa, Malassezia restricta, Malassezia furfur* or *Candida parapsilosis*
(iii) a fungus selected from *Aspergillus tubingensis, Zymoseptoria tritici, Tilletia walkeri, Epidermophyton floccosum, Nannizzia nana*,
(iv) a mite selected from *Demodex folliculorum, Demodex brevis.*

8. The method according to any of the preceding claims, wherein the biomarker of the microbiota is a protein exposed on the surface of the microorganism selected from Panton-Valentine leukocidin, porphyrins, alginates, β-1,6-linked N-acetylglucosamine (PNAG), Bap family adhesins, type 4 pili, self-associating autotransporters, intimins/invasin, CAMP factors, sialidases, dermatan-sulphate adhesins, endoglycoceramidases, GroEL chaperonins, SH3 domain-containing lipoprotein, Flp pili/fimbriae protein, DsA1 protein; Clf-Sdr family proteins, such as Bbp, FnBPs and CNA, SesJ protein, aureusimine, Bap family adhesins displaying an LPXTG C-terminal domain, IMPs, type 3 and 4 pili.

9. The method according to any of the preceding claims, wherein the skin cell biomarkers are selected from (i) a structural protein selected from keratin, filaggrin, loricrin, (ii) an inflammatory biomarker selected from C-reactive protein, interleukins IL-1beta, IL-4, IL-6, IL-8, IL-11, IL-12, tumor necrosis factor, interferon-gamma, granulocyte-macrophage colony-stimulating factor, transforming growth factor-beta and (iii) an allergy biomarker selected from immunoglobulins IgE, IgA and IgG.

10. The method according to any of the preceding claims, wherein the skin cell biomarker is interleukin 1-beta.

11. The method according to any of the preceding claims, **characterized in that** it comprises zones for specifically detecting the presence of bacteria of the genera *Staphylococcus, Cutibacterium* and *Corynebacterium* and of IL-1beta.

12. The method according to any of the preceding claims,
**characterized in that** the device is a lateral flow immunochromatographic device.

13. The method according to any of the preceding claims,
**characterized in that** the bacterial concentration of the solubilization solution after contact is made with the sampled biological material is approximately 10⁴ CFU/mL.
